# EUROPEAN PATENT APPLICATION

(11) **EP 4 831 181 A1**
(43) Date of publication of application: **28.10.2026**
(21) Application number: 24906519.4
(22) Date of filing: 20.12.2024
(51) Int. Cl.: C07D 495/14, C07D 495/12, A61P 25/28, A61P 25/18, A61P 25/22, A61K 31/381

(54) **SALT FORM OF TAAR1 LIGAND AGONIST, CRYSTAL FORM, PREPARATION METHOD, AND USE**

(30) Priority: 22.12.2023 CN 202311786100
(71) Applicant: Shandong Luye Pharmaceutical Co., Ltd., Yantai, Shandong 264670 (CN)
(72) Inventor: QU, Hao, Yantai, Shandong 264670 (CN); WU, Yanhua, Yantai, Shandong 264670 (CN); WANG, Guanghui, Yantai, Shandong 264670 (CN); ZHAO, Jiye, Yantai, Shandong 264670 (CN); LIAO, Wensheng, Yantai, Shandong 264670 (CN)
(74) Representative: karo IP
(86) International application number: PCT/CN2024/140925
(87) International publication number: WO 2025/131045

(57) **Abstract**

The present invention relates to a salt of a TAARI ligand agonist, a crystal form, a preparation method, and a use. Specifically, provided are a salt of a compound of formula I, a crystal form, a preparation method, and a use in the preparation of a drug for TAAR1-mediated related diseases.

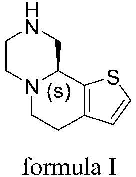

## Description

### TECHNICAL FIELD

The present disclosure belongs to the field of pharmaceutical chemistry and particularly relates to salts of a TAAR1 ligand agonist, crystal forms of the salts, preparation methods therefor, and use thereof.

### BACKGROUND

Trace amine-associated receptors (TAARs) are a family of endogenous biogenic amine-activated G protein-coupled receptors. Six TAARs and three pseudogenes have been identified in humans. TAAR1 is the most studied of all TAARs and is widely expressed in mammalian brains, particularly in the limbic and monoaminergic regions. TAAR1 can modulate presynaptic dopaminergic neurotransmission function, and can also induce the activation of G protein-coupled inwardly rectifying potassium ion channels and reduce neuronal firing frequency. TAAR1 may become a potential therapeutic target for treating psychiatric diseases such as schizophrenia, depression, Parkinson's disease, psychosis, behavioral and psychological symptoms of dementia, anxiety, migraine, and attention deficit hyperactivity disorder.

At present, there are 4 drugs acting on TAAR1: amphetamine, RG-7906, RO-5263397, and SEP-363856. Amphetamine was approved by the FDA for the treatment of attention deficit hyperactivity disorder in 2016. However, this compound has strong excitability to the central nervous system. Its short-term use may cause increased heart rates, increased blood pressure, feelings of happiness, and reduced fatigue, and its long-term use may cause adverse reactions such as insomnia, restlessness, delusions and hallucinations, and violent and assaultive actions; therefore, it has been classified as a controlled drug. RG-7906, RO-5263397, and SEP-363856, which are under development, are all TAAR1 selective agonists. RG-7906 is in Phase II clinical trials, RO-5263397 is in Phase I but the trials have been placed on clinical hold, and SEP-363856, which is the most advanced of them all, is in Phase III clinical trials. Therefore, there is a need to develop a new TAAR1 agonist with better safety and effectiveness to meet patients' needs. PCT/CN2023/101737 provides a TAAR1 ligand agonist with a structural formula as shown below. The test results show that the compound has good TAAR1 agonistic activity.

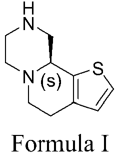

The pharmaceutical form of a compound, such as its crystal form or salt, often affects the chemical stability of the drug. Variations in crystallization and storage conditions may lead to changes in the crystal form structure of the compound and sometimes even result in the formation of other crystal forms. Generally, amorphous drug products lack a regular crystal form structure, which often leads to defects such as poor product stability, fine crystallization, filtration difficulties, proneness to agglomeration, and poor flowability. Given the importance of solid drug salt forms and crystal forms and their stability in clinical treatment, in-depth research on salt forms and crystal forms of the compound of Formula I is of great significance for the development of drugs that are suitable for industrial production and have good biological activity.

### SUMMARY

In one aspect, the present disclosure provides a pharmaceutically acceptable salt of the compound of Formula I, wherein the pharmaceutically acceptable salt is selected from the group consisting of citrate, succinate, maleate, fumarate, L-tartrate, gallate, mucate, phosphate, hydrochloride, oxalate, hydrobromide, sulfate, acetate, DL-lactate, ascorbate, hippurate, p-toluenesulfonate, and diphosphate.

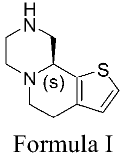

In some embodiments, the pharmaceutically acceptable salt is maleate, mucate, phosphate, and oxalate.

In some embodiments, the pharmaceutically acceptable salt is maleate, phosphate, and oxalate. In some embodiments, the pharmaceutically acceptable salt has a molar ratio of the compound of Formula I to an acid molecule of about 1:2 to 2:1, preferably about 1:2, 1:1, or 2:1.

In some embodiments, a molar ratio of the compound of Formula I to maleic acid is about 1:1. In some embodiments, a molar ratio of the compound of Formula I to phosphoric acid is about 1:1 or 1:2.

In one aspect, the present disclosure provides a preparation method for the pharmaceutically acceptable salt of the compound of Formula I, comprising a step of mixing the compound of Formula I with an acid and a solvent. The solvent is one or more selected from the group consisting of isopropanol, isopropyl acetate, toluene, and methyl tert-butyl ether.

In some embodiments, in a preparation method for the maleate salt of the compound of Formula I, a feeding amount molar ratio of maleic acid to the compound of Formula I is selected from the group consisting of 0.5:1 to 1.5:1, preferably 1:1.

In one aspect, the present disclosure provides a Crystal Form A of the maleate salt of the compound of Formula I, having, in an X-ray powder diffraction pattern using Cu-Kα radiation, characteristic diffraction peaks at the following 20 angles: 14.49±0.2°, 14.72±0.2°, 16.26±0.2°, 17.81±0.2°, 19.58±0.2°, 24.03±0.2°, 25.84±0.2°, and 29.72±0.2°.

In some embodiments, the Crystal Form A of the maleate salt of the compound of Formula I has, in an X-ray powder diffraction pattern using Cu-Kα radiation, characteristic diffraction peaks at the following 20 angles: 7.24+0.2°, 10.56+0.2°, 14.49+0.2°, 14.72+0.2°, 16.26+0.2°, 17.81±0.2°, 19.58+0.2°, 24.03+0.2°, 25.84+0.2°, and 29.72+0.2°.

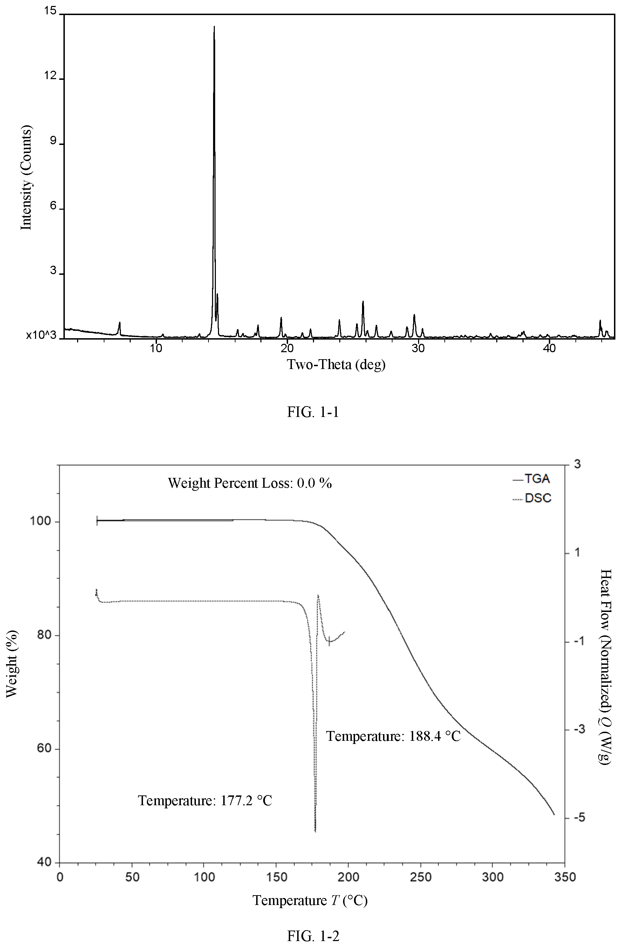

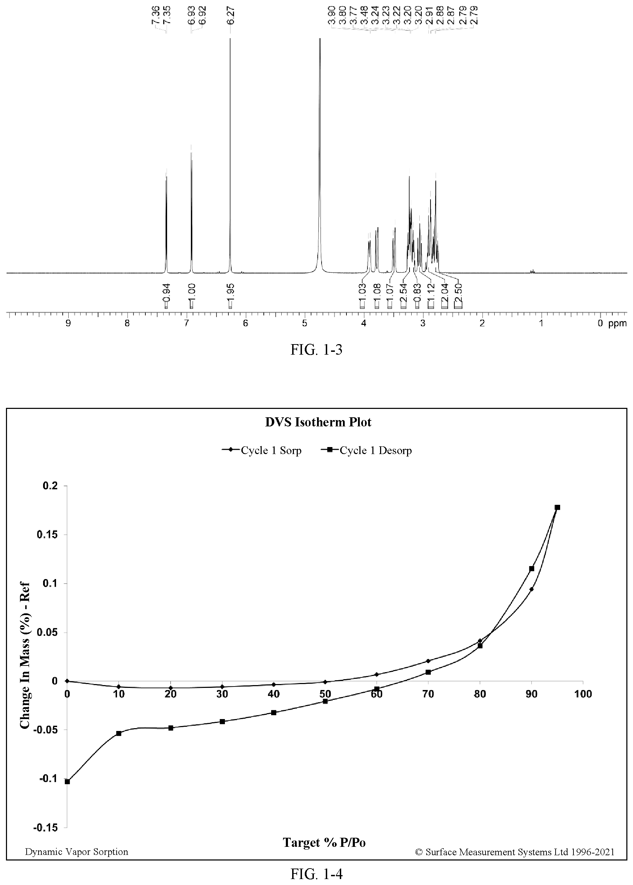

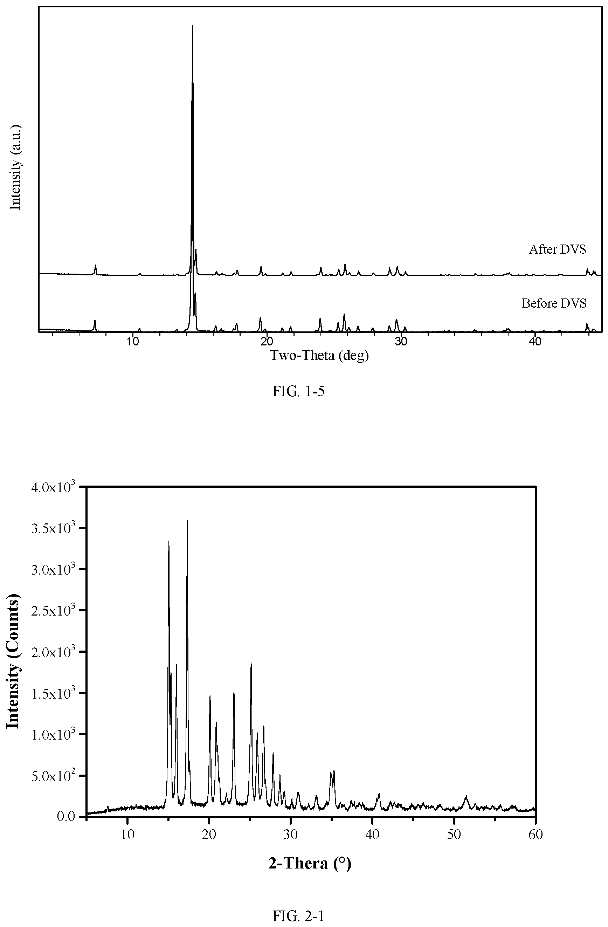

In some embodiments, the Crystal Form A of the maleate salt of the compound of Formula I has an X-ray powder diffraction pattern using Cu-Kα radiation as shown in FIG. 1-1.

In some embodiments, the Crystal Form A of the maleate salt of the compound of Formula I has a molar ratio of maleic acid to the compound of Formula I of about 1:1.

In an optional embodiment, the present disclosure provides a preparation method for the Crystal Form A of the maleate salt of the compound of Formula I, comprising a step of mixing the compound of Formula I with maleic acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form A of the phosphate salt of the compound of Formula I, having, in an X-ray powder diffraction pattern using Cu-Kα radiation, characteristic diffraction peaks at the following 2θ angles: 15.068±0.2°, 15.331±0.2°, 16.011±0.2°, 17.333±0.2°, 17.605±0.2°, 20.119±0.2°, 20.870±0.2°, 23.039±0.2°, 25.159±0.2°, 25.910±0.2°, 26.680±0.2°, and 27.843±0.2°.

In some embodiments, the Crystal Form A of the phosphate salt of the compound of Formula I has, in an X-ray powder diffraction pattern using Cu-Kα radiation, characteristic diffraction peaks at the following 2θ angles: 15.068±0.2°, 15.331±0.2°, 16.011±0.2°, 17.333±0.2°, 17.605±0.2°, 20.119±0.2°, 20.870±0.2°, 21.161±0.2°, 23.039±0.2°, 25.159±0.2°, 25.910±0.2°, 26.680±0.2°, 27.843±0.2°, 28.681±0.2°, 34.958±0.2°, and 35.302±0.2°.

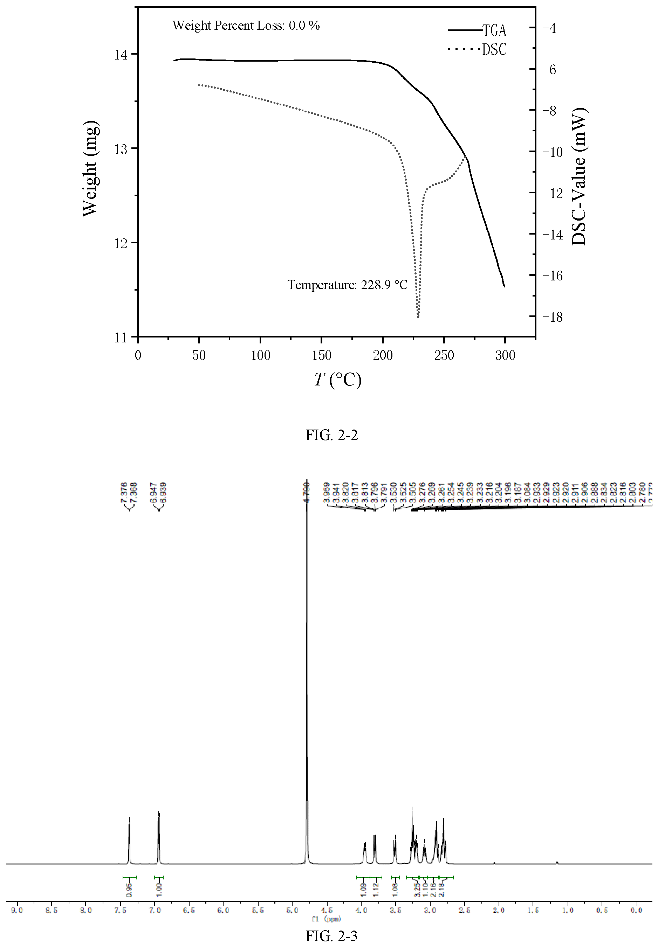

In some embodiments, the Crystal Form A of the phosphate salt of the compound of Formula I has an X-ray powder diffraction pattern using Cu-Kα radiation as shown in FIG. 2-1.

In an optional embodiment, the present disclosure provides a preparation method for the Crystal Form A of the phosphate salt of the compound of Formula I, comprising a step of mixing the compound of Formula I with phosphoric acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form A of the diphosphate salt of the compound of Formula I, having, in an X-ray powder diffraction pattern using Cu-Kα radiation, characteristic diffraction peaks at the following 20 angles: 11.029±0.2°, 12.607±0.2°, 14.362+0.2°, 14.849+0.2°, 15.635+0.2°, 18.139+0.2°, 19.187+0.2°, 20.687+0.2°, 21.808+0.2°, 22.432+0.2°, 22.715+0.2°, 23.294+0.2°, 23.533+0.2°, 24.283+0.2°, 24.788+0.2°, and 28.295+0.2°.

In some embodiments, the Crystal Form A of the diphosphate salt of the compound of Formula I has, in an X-ray powder diffraction pattern using Cu-Kα radiation, characteristic diffraction peaks at the following 20 angles: 7.839+0.2°, 11.029+0.2°, 12.607+0.2°, 14.362+0.2°, 14.849±0.2°, 15.635±0.2°, 17.454±0.2°, 18.139±0.2°, 19.187±0.2°, 19.773±0.2°, 20.369±0.2°, 20.687±0.2°, 21.049±0.2°, 21.808±0.2°, 22.432±0.2°, 22.715±0.2°, 23.294±0.2°, 23.533±0.2°, 24.283±0.2°, 24.788±0.2°, and 28.295±0.2°.

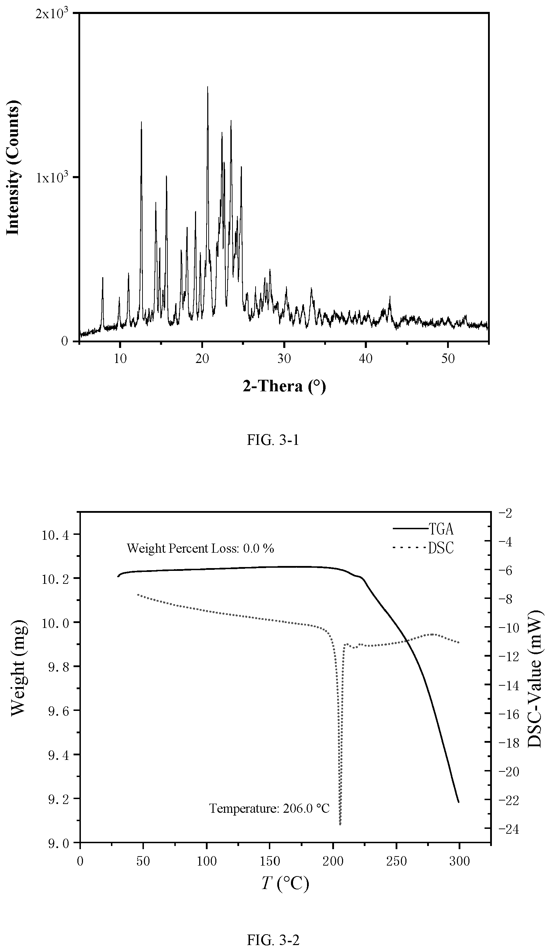

In some embodiments, the Crystal Form A of the diphosphate salt of the compound of Formula I has an X-ray powder diffraction pattern using Cu-Kα radiation as shown in FIG. 3-1.

In an optional embodiment, the present disclosure provides a preparation method for the Crystal Form A of the diphosphate salt of the compound of Formula I, comprising a step of mixing the compound of Formula I with phosphoric acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form A of the oxalate salt of the compound of Formula I, having, in an X-ray powder diffraction pattern using Cu-Kα radiation, characteristic diffraction peaks at the following 20 angles: 13.162±0.2°, 15.072±0.2°, 15.802±0.2°, 17.833±0.2°, 19.498±0.2°, 21.088±0.2°, 23.168±0.2°, 24.677±0.2°, 26.481±0.2°, 28.805±0.2°, and 32.226±0.2°.

In some embodiments, the Crystal Form A of the oxalate salt of the compound of Formula I has, in an X-ray powder diffraction pattern using Cu-Kα radiation, characteristic diffraction peaks at the following 2θ angles: 13.162±0.2°, 15.072±0.2°, 15.802±0.2°, 17.833±0.2°, 19.498±0.2°, 20.323±0.2°, 21.088±0.2°, 23.168±0.2°, 24.677±0.2°, 26.481±0.2°, 27.082±0.2°, 28.805±0.2°, 32.002±0.2°, and 32.226±0.2°.

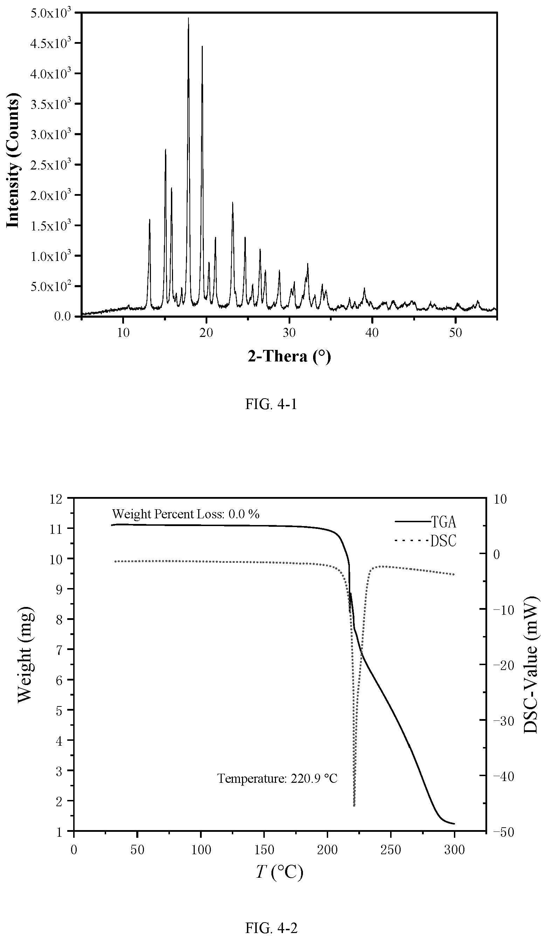

In some embodiments, the Crystal Form A of the oxalate salt of the compound of Formula I has an X-ray powder diffraction pattern using Cu-Kα radiation as shown in FIG. 4-1.

In an optional embodiment, the present disclosure provides a preparation method for the Crystal Form A of the oxalate salt of the compound of Formula I, comprising a step of mixing the compound of Formula I with oxalic acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form A of the mucate salt of the compound of Formula I, having, in an X-ray powder diffraction pattern using Cu-Kα radiation, characteristic diffraction peaks at the following 20 angles: 12.975+0.2°, 13.451+0.2°, 14.739+0.2°, 15.310+0.2°, 16.171±0.2°, 17.231+0.2°, 17.743+0.2°, 18.123+0.2°, 18.470+0.2°, 19.647+0.2°, 21.511+0.2°, 22.794+0.2°, 24.854+0.2°, 26.852+0.2°, 29.702+0.2°, 30.758+0.2°, 34.460+0.2°, 34.869+0.2°, 36.684+0.2°, and 37.671±0.2°.

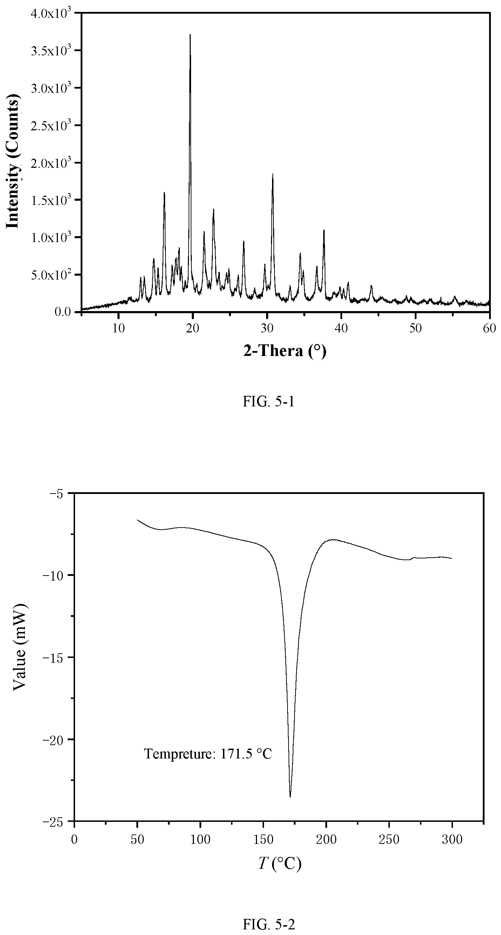

In some embodiments, the Crystal Form A of the mucate salt of the compound of Formula I has an X-ray powder diffraction pattern using Cu-Kα radiation as shown in FIG. 5-1.

In an optional embodiment, the present disclosure provides a preparation method for the Crystal Form A of the mucate salt of the compound of Formula I, comprising a step of mixing the compound of Formula I with mucic acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form A of the hydrobromide salt of the compound of Formula I, having, in an X-ray powder diffraction pattern using Cu-Kα radiation, characteristic diffraction peaks at the following 20 angles: 11.515±0.2°, 14.871±0.2°, 16.925±0.2°, 17.475±0.2°, 19.359±0.2°, 21.082±0.2°, 21.462±0.2°, 21.974±0.2°, 23.566±0.2°, 24.012±0.2°, 24.881±0.2°, 26.205±0.2°, 27.458±0.2°, 27.977±0.2°, 28.452±0.2°, 29.718±0.2°, 31.123±0.2°, 32.375±0.2°, 39.085±0.2°, and 42.023±0.2°.

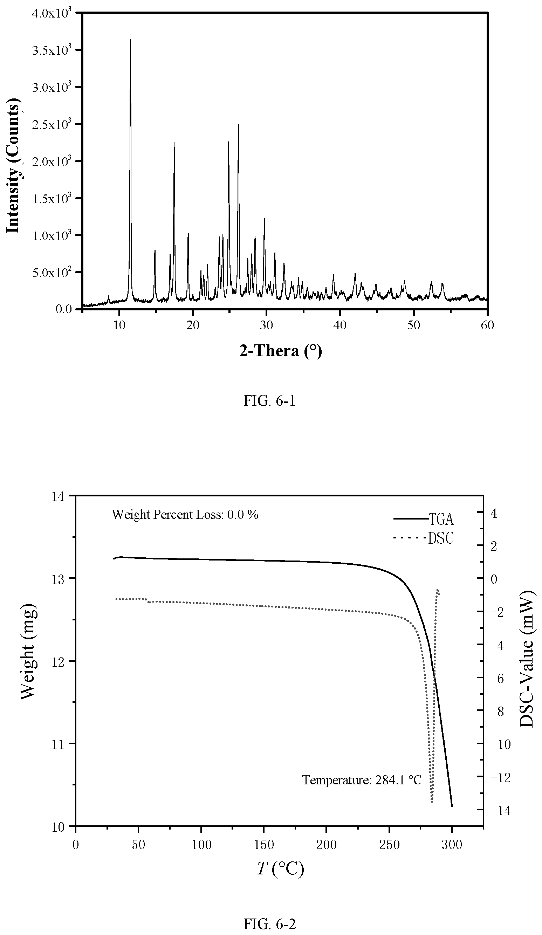

In some embodiments, the Crystal Form A of the hydrobromide salt of the compound of Formula I has an X-ray powder diffraction pattern using Cu-Kα radiation as shown in FIG. 6-1.

In some embodiments, the present disclosure provides a preparation method for the Crystal Form A of the hydrobromide salt of the compound of Formula I, comprising a step of mixing the compound of Formula I with hydrobromic acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form A of the fumarate salt of the compound of Formula I, having, in an X-ray powder diffraction pattern using Cu-Kα radiation, characteristic diffraction peaks at the following 20 angles: 10.588±0.2°, 13.403±0.2°, 14.031±0.2°, 15.720±0.2°, 16.342±0.2°, 20.889±0.2°, 20.927±0.2°, 21.435±0.2°, and 23.386±0.2°.

In some embodiments, the Crystal Form A of the fumarate salt of the compound of Formula I has, in an X-ray powder diffraction pattern using Cu-Kα radiation, characteristic diffraction peaks at the following 2θ angles: 10.588+0.2°, 13.403+0.2°, 14.031+0.2°, 15.720+0.2°, 16.342+0.2°, 20.213+0.2°, 20.889+0.2°, 20.927+0.2°, 21.435+0.2°, 23.386+0.2°, and 23.565+0.2°.

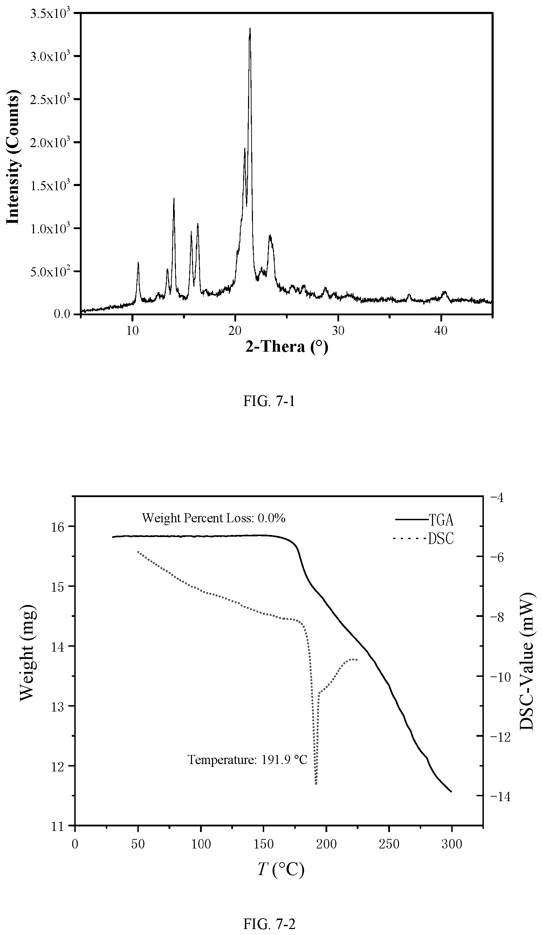

In some embodiments, the Crystal Form A of the fumarate salt of the compound of Formula I has an X-ray powder diffraction pattern using Cu-Kα radiation as shown in FIG. 7-1.

In some embodiments, the present disclosure provides a preparation method for the Crystal Form A of the fumarate salt of the compound of Formula I, comprising a step of mixing the compound of Formula I with fumaric acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form B of the fumarate salt of the compound of Formula I, having, in an X-ray powder diffraction pattern using Cu-Kα radiation, characteristic diffraction peaks at the following 2θ angles: 12.504±0.2°, 14.689±0.2°, 15.041±0.2°, 15.688±0.2°, 16.052±0.2°, 16.612±0.2°, 17.462±0.2°, 19.747±0.2°, 20.109±0.2°, 20.911±0.2°, 22.029±0.2°, 22.430±0.2°, 23.454±0.2°, 23.800±0.2°, 24.011±0.2°, 24.632±0.2°, 25.046±0.2°, 25.453±0.2°, 26.588±0.2°, and 27.589±0.2°.

In some embodiments, the Crystal Form B of the fumarate salt of the compound of Formula I has, in an X-ray powder diffraction pattern using Cu-Kα radiation, characteristic diffraction peaks at the following 2θ angles: 12.504±0.2°, 13.233±0.2°, 14.084±0.2°, 14.689±0.2°, 15.041±0.2°, 15.688±0.2°, 16.052±0.2°, 16.612±0.2°, 17.462±0.2°, 19.106±0.2°, 19.747±0.2°, 20.109±0.2°, 20.911±0.2°, 21.720±0.2°, 22.029±0.2°, 22.430±0.2°, 22.824±0.2°, 23.454±0.2°, 23.800±0.2°, 24.011±0.2°, 24.632±0.2°, 25.046±0.2°, 25.453±0.2°, 26.055±0.2°, 26.588±0.2°, 27.589±0.2°, 28.771±0.2°, 29.536±0.2°, 31.988±0.2°, 37.956±0.2°, and 40.993±0.2°.

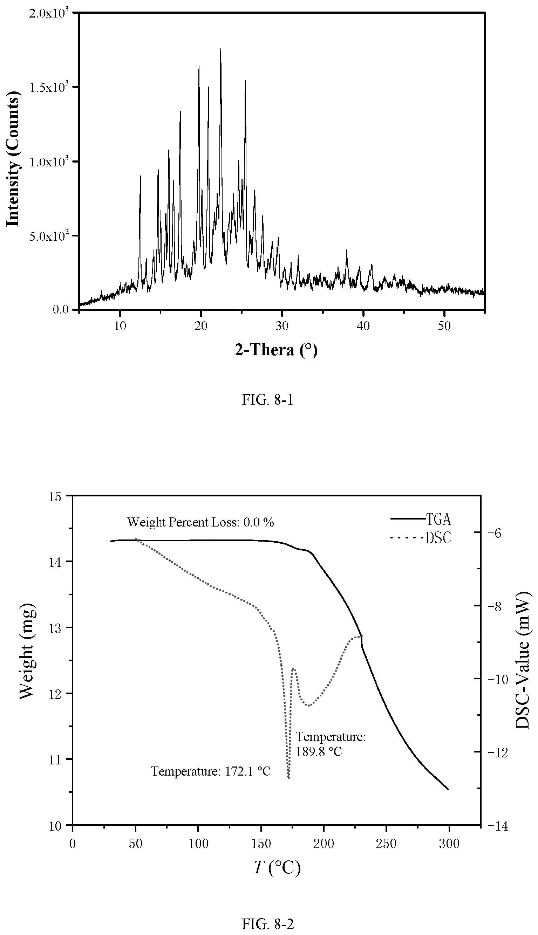

In some embodiments, the Crystal Form B of the fumarate salt of the compound of Formula I has an X-ray powder diffraction pattern using Cu-Kα radiation as shown in FIG. 8-1.

The present disclosure provides a preparation method for the Crystal Form B of the fumarate salt of the compound of Formula I, comprising a step of mixing the compound of Formula I with fumaric acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form A of the L-hemitartrate salt of the compound of Formula I, having, in an X-ray powder diffraction pattern using Cu-Kα radiation, characteristic diffraction peaks at the following 20 angles: 12.107±0.2°, 12.962±0.2°, 16.817+0.2°, 17.082+0.2°, 17.924+0.2°, 18.137+0.2°, 19.137+0.2°, 19.494+0.2°, 20.731+0.2°, 21.967+0.2°, 22.675+0.2°, 23.839+0.2°, 24.767+0.2°, 26.220+0.2°, 26.901±0.2°, 27.240+0.2°, 28.888+0.2°, and 37.559+0.2°.

In some embodiments, the Crystal Form A of the L-tartrate salt of the compound of Formula I has, in an X-ray powder diffraction pattern using Cu-Kα radiation, characteristic diffraction peaks at the following 2θ angles: 12.107+0.2°, 12.962+0.2°, 13.686+0.2°, 16.817+0.2°, 17.082±0.2°, 17.924±0.2°, 18.137±0.2°, 19.137±0.2°, 19.494±0.2°, 20.731±0.2°, 21.967±0.2°, 22.675±0.2°, 23.076±0.2°, 23.839±0.2°, 24.135±0.2°, 24.767±0.2°, 26.220±0.2°, 26.901±0.2°, 27.240±0.2°, 27.650±0.2°, 28.888±0.2°, 35.012±0.2°, 35.925±0.2°, 37.559±0.2°, 38.931±0.2°, and 39.157±0.2°.

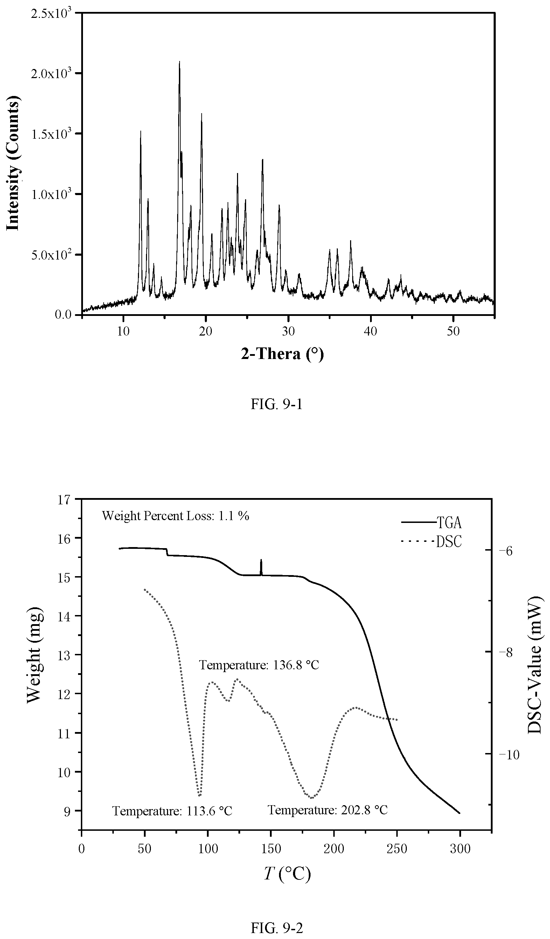

In some embodiments, the Crystal Form A of the L-tartrate salt of the compound of Formula I has an X-ray powder diffraction pattern using Cu-Kα radiation as shown in FIG. 9-1.

In some embodiments, the present disclosure provides a preparation method for the Crystal Form A of the L-tartrate salt of the compound of Formula I, comprising a step of mixing the compound of Formula I with L-tartaric acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form A of the gallate salt of the compound of Formula I, having, in an X-ray powder diffraction pattern using Cu-Kα radiation, characteristic diffraction peaks at the following 20 angles: 10.879±0.2°, 14.571±0.2°, 15.170±0.2°, 15.469±0.2°, 16.567±0.2°, 17.325±0.2°, 20.016±0.2°, 20.569±0.2°, 21.770±0.2°, 23.480±0.2°, 24.012±0.2°, 24.414±0.2°, 26.181±0.2°, 27.498±0.2°, and 27.850±0.2°.

In some embodiments, the Crystal Form A of the gallate salt of the compound of Formula I has, in an X-ray powder diffraction pattern using Cu-Kα radiation, characteristic diffraction peaks at the following 20 angles: 10.879±0.2°, 11.529±0.2°, 13.689±0.2°, 14.571±0.2°, 15.170±0.2°, 15.469±0.2°, 16.567±0.2°, 17.325±0.2°, 18.178±0.2°, 18.933±0.2°, 20.016±0.2°, 20.569±0.2°, 21.770±0.2°, 22.297±0.2°, 23.480±0.2°, 24.012±0.2°, 24.414±0.2°, 24.978±0.2°, 25.609±0.2°, 26.181±0.2°, 27.498±0.2°, 27.850±0.2°, 31.485±0.2°, 32.745±0.2°, and 34.343±0.2°.

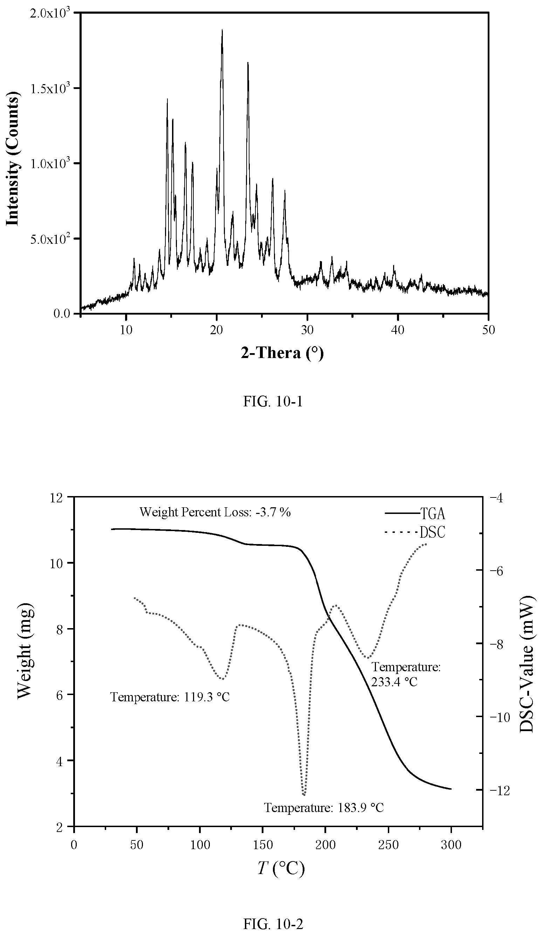

In some embodiments, the Crystal Form A of the gallate salt of the compound of Formula I has an X-ray powder diffraction pattern using Cu-Kα radiation as shown in FIG. 10-1.

In some embodiments, the present disclosure provides a preparation method for the Crystal Form A of the gallate salt of the compound of Formula I, comprising a step of mixing the compound of Formula I with gallic acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form A of the p-toluenesulfonate salt of the compound of Formula I, having, in an X-ray powder diffraction pattern using Cu-Kα radiation, characteristic diffraction peaks at the following 2θ angles: 7.845+0.2°, 13.526±0.2°, 14.080±0.2°, 17.124+0.2°, 19.848+0.2°, 20.209+0.2°, 20.780+0.2°, 23.534±0.2°, 25.368±0.2°, and 27.328±0.2°.

In some embodiments, the Crystal Form A of the p-toluenesulfonate salt of the compound of Formula I has, in an X-ray powder diffraction pattern using Cu-Kα radiation, characteristic diffraction peaks at the following 2θ angles: 7.845±0.2°, 13.526±0.2°, 14.080±0.2°, 15.692±0.2°, 16.451±0.2°, 17.124±0.2°, 19.848±0.2°, 20.209±0.2°, 20.780±0.2°, 23.534±0.2°, 24.627±0.2°, 25.368±0.2°, and 27.328±0.2°.

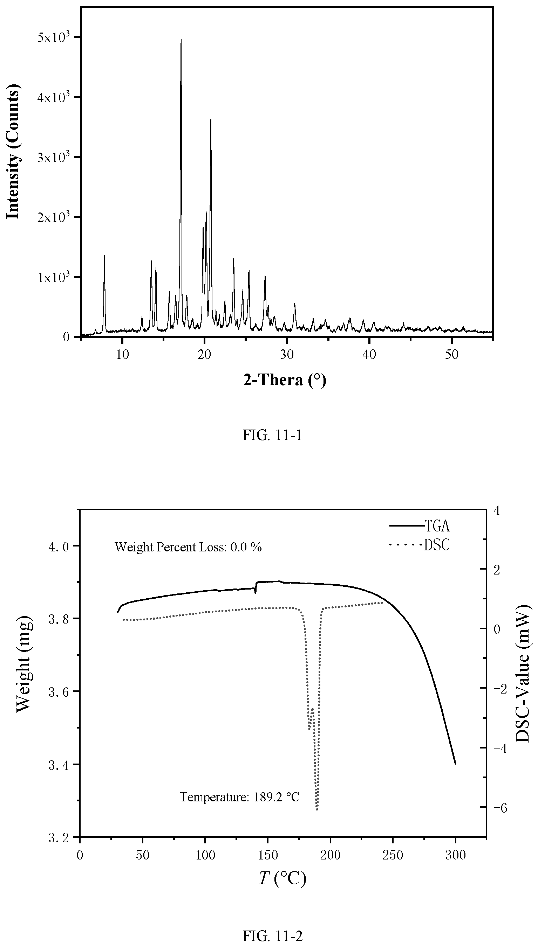

In some embodiments, the Crystal Form A of the p-toluenesulfonate salt of the compound of Formula I has an X-ray powder diffraction pattern using Cu-Kα radiation as shown in FIG. 11-1.

In some embodiments, the present disclosure provides a preparation method for the Crystal Form A of the p-toluenesulfonate salt of the compound of Formula I, comprising a step of mixing the compound of Formula I with p-toluenesulfonic acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In one aspect, the present disclosure provides a Crystal Form A of the hydrochloride salt of the compound of Formula I, having, in an X-ray powder diffraction pattern using Cu-Kα radiation, characteristic diffraction peaks at the following 2θ angles: 11.893±0.2°, 15.339±0.2°, 16.680±0.2°, 17.444±0.2°, 22.389±0.2°, 25.024±0.2°, 25.609±0.2°, 26.987±0.2°, and 28.243±0.2°.

In some embodiments, the Crystal Form A of the hydrochloride salt of the compound of Formula I has, in an X-ray powder diffraction pattern using Cu-Kα radiation, characteristic diffraction peaks at the following 2θ angles: 11.893±0.2°, 15.339±0.2°, 16.680±0.2°, 17.444±0.2°, 17.676±0.2°, 21.356±0.2°, 21.821±0.2°, 22.389±0.2°, 25.024±0.2°, 25.609±0.2°, 26.987±0.2°, 28.243±0.2°, 28.931±0.2°, and 31.150±0.2°.

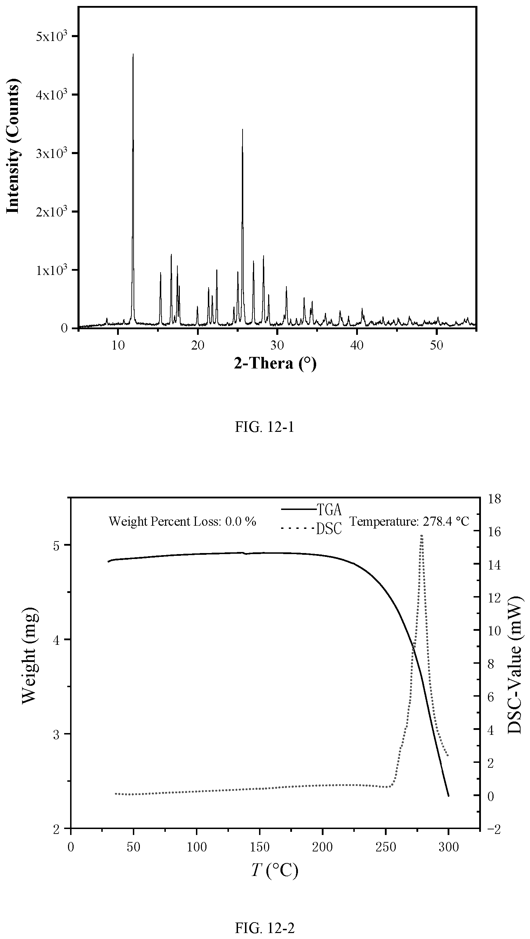

In some embodiments, the Crystal Form A of the hydrochloride salt of the compound of Formula I has an X-ray powder diffraction pattern using Cu-Kα radiation as shown in FIG. 12-1.

In some embodiments, the present disclosure provides a preparation method for the Crystal Form A of the hydrochloride salt of the compound of Formula I, comprising a step of mixing the compound of Formula I with hydrochloric acid and a solvent and a step of crystallization. In an optional embodiment, the solvent is selected from the group consisting of isopropanol, isopropyl acetate, toluene, and methyl tert-butyl ether. Modes of the crystallization include a volatilization method, a suspension method, an anti-solvent crystallization method, a cooling method, a gas-phase diffusion method, a ball-milling method, and a lyophilization method.

In some embodiments, the preparation method for any one of the crystal forms of the present disclosure further comprises steps such as filtration and drying.

The present disclosure further provides a pharmaceutical composition comprising any one of the aforementioned pharmaceutically acceptable salts of the compound of Formula I or the crystal forms thereof and a pharmaceutically acceptable carrier. The pharmaceutical composition may be prepared in various pharmaceutically acceptable dosage forms, such as tablets, capsules, oral liquids, granules, injections, or various sustained-release and controlled-release preparations. The pharmaceutical composition may be administered orally or parenterally (e.g., intravenously, subcutaneously, or topically). The administration dose may be appropriately adjusted based on the patient's age, sex, and disease type, and the daily dose is generally about 1-200 mg.

In one aspect, the present disclosure further provides use of any one of the aforementioned pharmaceutically acceptable salts or crystal forms of the compound of Formula I or the pharmaceutical composition in the preparation of a TAAR1 agonist medicament.

In one aspect, the present disclosure further provides use of any one of the aforementioned pharmaceutically acceptable salts or crystal forms of the compound of Formula I or the pharmaceutical composition in the preparation of a medicament for preventing and/or controlling central nervous system (CNS)-related diseases or disorders. The central nervous system (CNS) diseases or disorders include schizophrenia, schizophrenia spectrum disorder, acute schizophrenia, chronic schizophrenia, NOS schizophrenia, psychotic disorder, schizoid personality disorder, schizotypal personality disorder, delusional disorder, psychosis, mental disorder, brief psychotic disorder, shared psychotic disorder, psychotic disorder due to a physical disease, drug-induced psychosis, psychoaffective disorder, aggressive delirium, Parkinson's psychosis, excitative psychosis, Tourette syndrome, organic or NOS psychosis, epilepsy, epileptic seizures, agitation, post-traumatic stress disorder, behavioral disorder, neurodegenerative disease, Alzheimer's disease, Parkinson's disease, dyskinesias, Huntington's disease, dementia, affective disorder, anxiety, affective psychosis, obsessive compulsive disorder, vertigo, pain, fibromyalgia, migraine, cognitive impairment, movement disorder, restless leg syndrome (RLS), multiple sclerosis, psychoactive substance abuse, and stress-related disorders. The affective psychosis includes depression, major depressive disorder and dysthymia, bipolar disorder, bipolar depression, manic disorder, seasonal affective psychosis, attention deficit disorder (ADD), and attention deficit hyperactivity disorder (ADHD); the pain includes neuropathic pain, neuropathic pain susceptibility state, and inflammatory pain; the stress-related disorders include: acute stress disorder, post-traumatic stress disorder, and adjustment disorder. In one aspect, the present disclosure further provides use of any one of the aforementioned pharmaceutically acceptable salts or crystal forms of the compound of Formula I or the pharmaceutical composition in the preparation of a medicament for preventing and/or controlling cardiovascular or metabolic diseases. The cardiovascular or metabolic diseases include diabetes, diabetic complications, obesity, dyslipidemia, and hypertension.

### Definitions and Explanations

Unless otherwise specified, the following terms and phrases used herein are intended to have the following meanings. A particular term or phrase, when not specifically defined, should not be considered indefinite or unclear, but should be construed according to its common meaning. When referring to a trade name, it is intended to refer to its corresponding commercial product or its active ingredient.

The term "pharmaceutically acceptable" is described in the present disclosure in terms of compounds, compositions, and/or dosage forms that are, within the scope of sound medical judgment, suitable for contact with tissues of humans and animals without undue toxicity, irritation, allergic response, or other problems or complications, and commensurate with a reasonable benefit/risk ratio.

The compounds of the present disclosure may have asymmetric carbon atoms (optical centers) or double bonds. Racemates, diastereoisomers, geometric isomers, and individual isomers all fall within the scope of the present disclosure.

The compounds of the present disclosure may be present in particular geometric or stereoisomeric forms. All such compounds are contemplated herein, including cis and trans isomers, (-)- and (+)-enantiomers, (R)- and (S)-enantiomers, diastereoisomers, (D)-isomers, (L)-isomers, and racemic mixtures and other mixtures thereof, such as enantiomerically or diastereoisomerically enriched mixtures, all of which fall within the scope of the present disclosure. Additional asymmetric carbon atoms may be present in substituents such as alkyl. All such isomers and mixtures thereof fall within the scope of the present disclosure.

The term "pharmaceutically acceptable carrier" refers to any preparation or carrier medium that is capable of delivering an effective amount of the active substance of the present disclosure, does not interfere with the biological activity of the active substance, and has no toxic or side effects on a host or patient; representative carriers include, but are not limited to: binders, fillers, lubricants, disintegrants, wetting agents, dispersing agents, solubilizers, suspending agents, etc. The present disclosure is intended to include all isotopes of atoms present in the compounds of the present disclosure. Isotopes include those atoms having the same atomic number but different mass numbers. As a general and non-limiting example, isotopes of hydrogen include deuterium and tritium. Isotopes of carbon include ¹³C and ¹⁴C. Isotopically labeled compounds of the present disclosure can generally be prepared by conventional techniques known to those skilled in the art or by methods similar to those described herein using an appropriate isotopically labeled reagent in place of the non-labeled reagent otherwise used.

### BRIEF DESCRIPTION OF THE DRAWINGS

FIGs. 1-1, 1-2, 1-3, 1-4, and 1-5 show an XRPD pattern, TGA/DSC thermograms, a ¹H NMR spectrum, a DVS isotherm plot, and XRPD patterns before and after DVS testing of the Crystal Form A of the maleate salt of the compound of Formula I, respectively.
FIGs. 2-1, 2-2, and 2-3 show an XRPD pattern, TGA/DSC thermograms, and a ¹H NMR spectrum of the Crystal Form A of the phosphate salt of the compound of Formula I, respectively.
FIGs. 3-1 and 3-2 show an XRPD pattern and TGA/DSC thermograms of the Crystal Form A of the diphosphate salt of the compound of Formula I, respectively.
FIGs. 4-1 and 4-2 show an XRPD pattern and TGA/DSC thermograms of the Crystal Form A of the oxalate salt of the compound of Formula I, respectively.
FIGs. 5-1 and 5-2 show an XRPD pattern and a DSC thermogram of the Crystal Form A of the mucate salt of the compound of Formula I, respectively.
FIGs. 6-1 and 6-2 show an XRPD pattern and TGA/DSC thermograms of the Crystal Form A of the hydrobromide salt of the compound of Formula I, respectively.
FIGs. 7-1 and 7-2 show an XRPD pattern and TGA/DSC thermograms of the Crystal Form A of the fumarate salt of the compound of Formula I, respectively.
FIGs. 8-1 and 8-2 show an XRPD pattern and TGA/DSC thermograms of the Crystal Form B of the fumarate salt of the compound of Formula I, respectively.
FIGs. 9-1 and 9-2 show an XRPD pattern and TGA/DSC thermograms of the Crystal Form A of the L-tartrate salt of the compound of Formula I, respectively.
FIGs. 10-1 and 10-2 show an XRPD pattern and TGA/DSC thermograms of the Crystal Form A of the gallate salt of the compound of Formula I, respectively.
FIGs. 11-1 and 11-2 show an XRPD pattern and TGA/DSC thermograms of the Crystal Form A of the p-toluenesulfonate salt of the compound of Formula I, respectively.
FIGs. 12-1 and 12-2 show an XRPD pattern and TGA/DSC thermograms of the Crystal Form A of the hydrochloride salt of the compound of Formula I, respectively.
FIG. 13 shows XRPD patterns of the Crystal Form A of the maleate salt of the compound of Formula I under high temperature (60 °C), high humidity (25 °C/92.5% RH), light exposure (25 °C/4500 Lux), and accelerated (40 °C/75% RH) conditions.

### DETAILED DESCRIPTION

The present disclosure is further illustrated below with reference to specific examples and test examples, but these examples do not limit the scope of the present disclosure in any way.

Test conditions for the instruments used in the experiments:

### X-ray powder diffraction (XRPD)

Samples were analyzed using a Bruker D8 Advance X-ray powder diffractometer (Bruker, GER), and X-ray powder diffraction data of the samples were collected under ambient conditions. The 20 scanning angle was from 3° to 45°, the scanning step size was 0.02°, and the exposure time was 0.08 s. The test method was Cu target Kα1 radiation at a voltage of 40 kV and a current of 40 mA, and a zero-background sample pan was used. The current was 40 mA, and a zero-background sample pan was used.

### Thermogravimetric analyzer (TGA)

The thermogravimetric analyzer model was TA Discovery 55 (TA, US). A 2-5 mg sample was placed in an equilibrated open aluminum sample pan and automatically weighed in a TGA furnace. The sample was heated to the final temperature at a rate of 10 °C/min with a nitrogen purging speed of 60 mL/min at the sample and a nitrogen purging speed of 40 mL/min at the balance.

### Differential scanning calorimeter (DSC)

The differential scanning calorimeter model was TA Discovery 2500 (TA, US). A 1-2 mg sample was accurately weighed out, then placed in a perforated DSC Tzero sample pan, and heated to the final temperature at a rate of 10 °C/min with a nitrogen purging speed of 50 mL/min in the furnace.

### Proton nuclear magnetic resonance spectroscopy (1H NMR)

Solid samples were dissolved in D₂O (solvent) and analyzed using a Bruker AVANCE NEO 400 nuclear magnetic resonance spectrometer (Bruker, GER).

### Dynamic vapor sorption (DVS)

Dynamic vapor sorption analysis was performed on a DVS Intrinsic Plus instrument (SMS, UK). The test employed a gradient mode. The humidity was changed from 0% to 95% and back to 0%. The humidity change for each gradient was 10% in the range of 0% to 90%. The gradient endpoint was judged using dm/dt and was defined as a dm/dt of less than 0.002% lasting for 10 min, or each gradient lasting for a maximum of 180 min. After the test was completed, the sample was analyzed by XRPD to determine whether the solid form had changed.

### Example 1. Synthesis of Compound of Formula I

### 1.1. Synthesis of intermediate 1-2

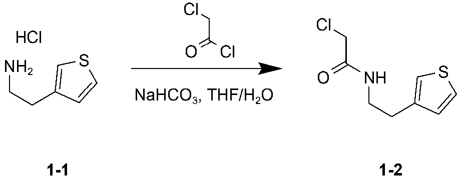

Compound 1-1 (5.00 g, 30.6 mmol, 1.00 eq) and NaHCO₃ (5.65 g, 67.2 mmol, 2.61 mL, 2.20 eq) were dissolved in THF (100 mL) and water (10.0 mL). The solution was cooled to 0-5 °C, and chloroacetyl chloride (5.31 g, 47.05 mmol, 1.10 eq) was added dropwise. After the dropwise addition, the mixture was naturally warmed to 20 °C and stirred for 24 h. NaHCO3 (2.57 g, 30.6 mmol, 1.00 eq) and the compound chloroacetyl chloride (3.45 g, 30.6 mmol, 1.00 eq) were additionally added. After the addition, the reaction solution was stirred at 20 °C for 48 h. LCMS analysis showed that the reaction had been complete. The reaction solution was concentrated under reduced pressure to give a crude product, and the crude product was dissolved in EtOAc (150 mL) and water (100 mL). The organic phase was isolated, and the aqueous phase was extracted once with EtOAc (100 mL). The organic phases were combined, washed once with brine (50.0 mL), dried over anhydrous Na₂SO₄, and concentrated under reduced pressure to give a crude product. The crude product was purified by silica gel column chromatography (petroleum ether:ethyl acetate = 5:1) and concentrated to dryness to give compound 1-2 (3.00 g, 13.6 mmol, yield: 44.4%) as a white solid.

### 1.2. Synthesis of intermediate 1-3

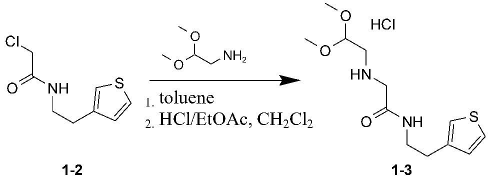

Compound 1-2 (3.00 g, 14.7 mmol, 1.00 eq) and aminoacetaldehyde dimethyl acetal (3.25 g, 30.9 mmol, 3.37 mL, 2.10 eq) were dissolved in anhydrous toluene (30.0 mL), and the solution was heated to 115 °C and stirred for 2 h. LCMS analysis showed that the reaction had been complete. The reaction solution was cooled to 0-5 °C, and a large amount of solid precipitated. The mixture was filtered, and the filter cake was washed with EtOAc (30.0 mL). The filtrate was washed with brine (30.0 mL), dried over anhydrous Na₂SO₄, and concentrated under reduced pressure until about 30.0 mL of solvent remained. The remaining organic phase was cooled to 0-5 °C. HCl/EtOAc (4 M) was added dropwise to adjust the pH to 2-3, and a large amount of solid precipitated. The mixture was filtered. The filter cake was washed with EtOAc (5.00 mL) and dried under reduced pressure, and the solid was collected. The solid was concentrated under reduced pressure to remove the solvent, affording compound 1-3 (3.20 g, 9.78 mmol, yield: 66.4%) as an off-white solid.

### 1.3. Synthesis of intermediate 1-4

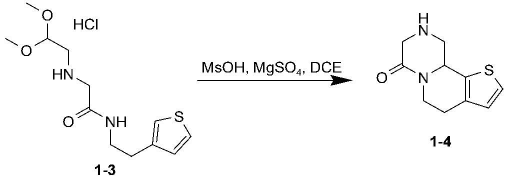

Compound 1-3 (2.63 g, 8.52 mmol, 1.00 eq) was suspended in DCE (130 mL), and MgSO4 (2.05 g, 17.0 mmol, 2.00 eq) and MsOH (4.91 g, 51.1 mmol, 3.64 mL, 6.00 eq) were added. The reaction solution was heated to 85 °C and stirred for 3 h. LCMS analysis showed that compound 3 had been completely reacted. The reaction solution was cooled to 20 °C. Water (100 mL) was added, and the pH was adjusted to 9-10 with an aqueous Na₂CO₃ solution. Insoluble salts were removed by filtration, and the filter cake was washed with CH₂Cl₂ (150 mL) and water (100 mL). The filtrate was separated, and the aqueous phase was extracted twice with CH₂Cl₂ (150 mL). The organic phases were combined, washed with brine (50.0 mL), dried over anhydrous Na₂SO₄, and concentrated to dryness under reduced pressure to give compound 1-4 (crude, 2.00 g) as a white solid.

### 1.4. Synthesis of compound of Formula I

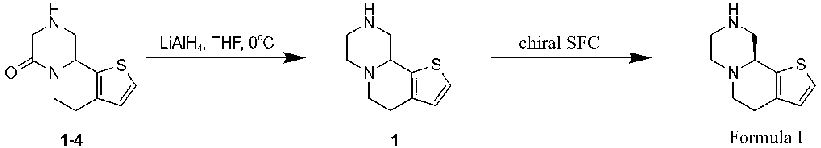

Compound 1-4 (1.49 g, 7.15 mmol, 1.00 eq) was dissolved in anhydrous THF (31 mL), and LiAlH₄ (543 mg, 14.3 mmol, 2.00 eq) was added in portions at 20 °C. The reaction was heated to 66 °C and stirred for 1 h. A product was detected by LCMS. Water (1.50 mL) was added dropwise to the reaction solution in a nitrogen atmosphere to quench the reaction, and the reaction solution was concentrated to dryness by rotary evaporation. The crude product was purified by silica gel column chromatography (dichloromethane:methanol = 100:0 to 96:4) to give a yellow oily substance. The product was resolved by preparative chromatography and lyophilized to give compound 1. The product was subjected to chiral resolution (column: DAICEL CHIRALPAK AY-H (250 mm × 30 mm, 10 µm); mobile phase: A: CO₂, B:(0.1% NH₃H₂O EtOH); B%: 30%-30%, 4 min) to give the compound of Formula I (retention time: 1.368 min, 275 mg, 1.39 mmol, yield: 19.4%) as a white solid. ¹H NMR: (400 MHz CDCl3) δ 7.11 (d, J = 5.2 Hz, 1H), 6.80 (d, J = 5.2 Hz, 1H), 3.36-3.29 (m, 2H), 3.07-2.90 (m, 5H), 2.83-2.80 (m, 1H), 2.66-2.62 (m, 2H), 2.54-2.48 (m, 1H). MS (ESI) m/z = 195.1 [M+H]⁺.

### Example 2. Crystal Form A of Maleate Salt of Compound of Formula I

About 1 g of the compound of Formula I was weighed out and added to a reaction flask, and 10 mL of isopropanol was added. After complete dissolution, 1 equivalent of maleic acid was added. The mixture was left to react at room temperature, and crystallization was monitored in real time. After a solid precipitated, the mixture was left to react for 30 min with the temperature maintained at 25 °C, then heated to 50 °C and stirred for 2 h with the temperature maintained, and then cooled to 25 °C and stirred overnight. The reaction solution was filtered, and the filter cake was washed and dried to give the Crystal Form A of the maleate salt of the compound of Formula I (1.44 g, yield: 90.1%, chemical purity: 99.86%). The XRPD pattern (FIG. 1-1) shows relatively high crystallinity. The characteristic peak positions are shown in Table 1. The TGA result (FIG. 1-2) shows that there was nearly no weight loss during heating from room temperature to 120 °C. The DSC result (FIG. 1-2) shows a melting endothermic peak at about 177 °C and an endothermic signal at about 188 °C. The ¹H NMR result (FIG. 1-3) shows that the Crystal Form A of the maleate salt of the compound of Formula I had a molar ratio of maleic acid to the compound of Formula I of 1:1, and there was no significant organic solvent peak. The DVS result (FIG. 1-4) shows that at a constant temperature of 25 °C, the Crystal Form A of the maleate salt of the compound of Formula I had an adsorption weight increase of about 0.18% at 95% RH, an adsorption weight increase of about 0.03% and a desorption weight increase of 0.03% at 80% RH, and a desorption weight loss of about 0.10% at 0% RH, indicating that the crystal form showed nearly no hygroscopicity. The XRPD results (FIG. 1-5) before and after DVS testing show that the crystal form of the sample did not change. According to the above characterization results, it can be seen that the Crystal Form A of the maleate salt was an anhydrous crystal form. When the amount of maleic acid was 0.5-1.5 equivalents, the Crystal Form A of the maleate salt could be obtained, with a molar ratio of maleic acid to the compound of Formula I of about 1:1.

**Table 1. XRPD pattern analysis data for the Crystal Form A of the maleate salt of the compound of Formula I**

| No. | 2θ±0.2 (°) | Relative intensity (%) | No. | 2θ±0.2 (°) | Relative intensity (%) |
|---|---|---|---|---|---|
| 1 | 7.24 | 6.9 | 23 | 27.96 | 5.3 |
| 2 | 10.56 | 5.0 | 24 | 29.18 | 4.5 |
| 3 | 12.29 | 1.9 | 25 | 29.72 | 10.9 |
| 4 | 13.34 | 3.0 | 26 | 30.37 | 4.2 |
| 5 | 14.49 | 100.0 | 27 | 32.81 | 2.2 |
| 6 | 14.72 | 29.2 | 28 | 33.01 | 2.4 |
| 7 | 16.26 | 7.1 | 29 | 33.63 | 2.9 |
| 8 | 16.66 | 4.0 | 30 | 34.62 | 2.0 |
| 9 | 17.58 | 3.3 | 31 | 35.56 | 2.9 |
| 10 | 17.81 | 9.7 | 32 | 36.02 | 2.3 |
| 11 | 19.58 | 13.1 | 33 | 36.90 | 2.0 |
| 12 | 19.91 | 5.4 | 34 | 37.71 | 3.4 |
| 13 | 21.19 | 6.5 | 35 | 37.97 | 3.2 |
| 14 | 21.83 | 5.1 | 36 | 38.10 | 2.9 |
| 15 | 23.74 | 2.9 | 37 | 39.36 | 2.1 |
| 16 | 24.03 | 19.0 | 38 | 39.91 | 2.3 |
| 17 | 24.71 | 2.6 | 39 | 40.76 | 2.2 |
| 18 | 24.88 | 2.3 | 40 | 41.97 | 2.1 |
| 19 | 25.37 | 5.9 | 41 | 43.91 | 4.8 |
| 20 | 25.84 | 18.3 | 42 | 44.03 | 3.3 |
| 21 | 26.19 | 6.4 | 43 | 44.44 | 2.4 |
| 22 | 26.85 | 5.6 | - | - | - |

### Example 3. Crystal Form A of Phosphate Salt of Compound of Formula I

About 1 g of the compound of Formula I was weighed out and added to a reaction flask, and 8 mL of isopropanol was added. After complete dissolution, the solution was heated to 50 °C, and a mixed solution prepared from 1 equivalent of phosphoric acid and 2 mL of isopropanol was added dropwise. After the dropwise addition, the mixture was left to react for 30 min with the temperature maintained, then heated to 80 °C and stirred for 1 h with the temperature maintained, and then cooled to 25 °C and stirred overnight. The reaction solution was filtered, and the filter cake was washed and dried to give the Crystal Form A of the phosphate salt of the compound of Formula I as an off-white solid (1.42 g, yield: 94.4%, chemical purity: 99.19%). The XRPD pattern (FIG. 2-1) shows relatively high crystallinity. The characteristic peak positions are shown in Table 2. The TGA result (FIG. 2-2) shows that there was nearly no weight loss during heating from room temperature to 120 °C. The DSC result (FIG. 2-2) shows a melting endothermic peak at 228.9 °C (peak temperature). The ¹H NMR result (FIG. 2-3) shows no significant organic solvent peak for the Crystal Form A of the phosphate salt of the compound of Formula I. According to the above characterization results, it can be seen that the Crystal Form A of the phosphate salt was an anhydrous crystal form.

**Table 2. XRPD pattern analysis data for the Crystal Form A of the phosphate salt of the compound of Formula I**

| No. | 2θ±0.2 (°) | Relative intensity (%) | No. | 2θ±0.2 (°) | Relative intensity (%) |
|---|---|---|---|---|---|
| 1 | 7.597 | 1.6 | 23 | 34.958 | 11.9 |
| 2 | 15.068 | 93.1 | 24 | 35.302 | 13.1 |
| 3 | 15.331 | 47.0 | 25 | 36.095 | 2.2 |
| 4 | 16.011 | 49.2 | 26 | 37.388 | 3.1 |
| 5 | 17.333 | 100.0 | 27 | 37.772 | 2.6 |
| 6 | 17.605 | 15.0 | 28 | 38.409 | 1.9 |
| 7 | 20.119 | 38.2 | 29 | 38.790 | 2.3 |
| 8 | 20.870 | 28.8 | 30 | 40.511 | 3.7 |
| 9 | 21.161 | 8.7 | 31 | 40.801 | 4.9 |
| 10 | 22.124 | 3.6 | 32 | 42.175 | 2.8 |
| 11 | 23.039 | 39.2 | 33 | 42.698 | 2.2 |
| 12 | 25.159 | 49.4 | 34 | 44.860 | 1.3 |
| 13 | 25.910 | 23.4 | 35 | 45.604 | 1.2 |
| 14 | 26.680 | 27.6 | 36 | 46.232 | 1.8 |
| 15 | 27.843 | 18.6 | 37 | 47.294 | 1.0 |
| 16 | 28.681 | 11.1 | 38 | 48.245 | 1.2 |
| 17 | 29.186 | 5.7 | 39 | 49.921 | 0.9 |
| 18 | 30.128 | 2.4 | 40 | 51.456 | 3.7 |
| 19 | 30.912 | 4.9 | 41 | 52.623 | 1.6 |
| 20 | 32.195 | 1.3 | 42 | 54.708 | 1.2 |
| 21 | 33.117 | 4.2 | 43 | 55.701 | 2.0 |
| 22 | 34.420 | 2.4 | | | |

### Example 4. Crystal Form A of Diphosphate Salt of Compound of Formula I

About 0.3 g of the compound of Formula I was weighed out and added to a reaction flask, and 1 mL of isopropanol was added. After complete dissolution, the solution was heated to 50 °C, and a mixed solution prepared from 6.0 equivalents of phosphoric acid and 1 mL of isopropanol was added dropwise. After the dropwise addition, the mixture was left to react for 30 min with the temperature maintained, then heated to 80 °C and stirred for 1 h with the temperature maintained, and then cooled to 25 °C and stirred overnight. The reaction solution was filtered, and the filter cake was washed and dried to give the Crystal Form A of the diphosphate salt of the compound of Formula I as an off-white solid (0.6 g, yield: 100%). The XRPD pattern (FIG. 3-1) shows relatively high crystallinity. The characteristic peak positions are shown in Table 3. The TGA result (FIG. 3-2) shows that there was nearly no weight loss during heating from room temperature to 120 °C. The DSC result (FIG. 3-2) shows a melting endothermic peak at 206.0 °C (peak temperature).

**Table 3. XRPD pattern analysis data for the Crystal Form A of the diphosphate salt of the compound of Formula I**

| No. | 2θ±0.2 (°) | Relative intensity (%) | No. | 2θ±0.2 (°) | Relative intensity (%) |
|---|---|---|---|---|---|
| 1 | 7.839 | 18.9 | 31 | 27.661 | 17.1 |
| 2 | 9.891 | 12.4 | 32 | 27.931 | 15.0 |
| 3 | 11.029 | 23.3 | 33 | 28.295 | 22.0 |
| 4 | 12.607 | 90.9 | 34 | 29.137 | 6.0 |
| 5 | 13.075 | 4.2 | 35 | 30.289 | 14.8 |
| 6 | 13.540 | 5.5 | 36 | 30.529 | 7.1 |
| 7 | 13.916 | 5.4 | 37 | 30.855 | 4.4 |
| 8 | 14.362 | 51.1 | 38 | 31.546 | 7.7 |
| 9 | 14.849 | 32.2 | 39 | 32.347 | 9.3 |
| 10 | 15.238 | 14.6 | 40 | 33.355 | 14.7 |
| 11 | 15.635 | 65.8 | 41 | 33.635 | 10.0 |
| 12 | 16.762 | 8.1 | 42 | 34.348 | 6.0 |
| 13 | 17.454 | 28.2 | 43 | 35.072 | 3.9 |
| 14 | 18.139 | 38.9 | 44 | 35.885 | 3.5 |
| 15 | 19.187 | 47.8 | 45 | 36.140 | 5.2 |
| 16 | 19.773 | 29.1 | 46 | 37.029 | 3.1 |
| 17 | 20.369 | 24.6 | 47 | 37.981 | 4.1 |
| 18 | 20.687 | 100.0 | 48 | 38.698 | 4.6 |
| 19 | 21.049 | 23.8 | 49 | 39.220 | 4.3 |
| 20 | 21.808 | 31.6 | 50 | 39.835 | 3.2 |
| 21 | 22.432 | 79.8 | 51 | 40.269 | 4.8 |
| 22 | 22.715 | 65.8 | 52 | 42.047 | 6.5 |
| 23 | 23.294 | 39.6 | 53 | 42.234 | 5.1 |
| 24 | 23.533 | 86.1 | 54 | 42.889 | 12.0 |
| 25 | 24.283 | 41.6 | 55 | 49.246 | 2.3 |
| 26 | 24.788 | 66.7 | 56 | 50.017 | 2.8 |
| 27 | 25.527 | 10.7 | 57 | 51.072 | 2.2 |
| 28 | 26.071 | 3.8 | 58 | 52.109 | 3.7 |
| 29 | 26.530 | 12.4 | 59 | 55.230 | 2.6 |
| 30 | 27.168 | 9.9 | | | |

### Example 5. Crystal Form A of Oxalate Salt of Compound of Formula I

200 mg of the compound of Formula I and 1.1 equivalents of oxalic acid were weighed out and placed into a 10 mL vial, and a certain amount of isopropanol was added. The reaction solution was stirred at room temperature (it gradually became viscous), heated to 70 °C and stirred for 1 h, and then cooled to room temperature and stirred overnight. The reaction solution was filtered, and the filter cake was washed and dried to give the Crystal Form A of the oxalate salt of the compound of Formula I (257 mg). The XRPD pattern (FIG. 4-1) shows relatively high crystallinity. The characteristic peak positions are shown in Table 4. The DSC result (FIG. 4-2) shows an endothermic signal at 220.9 °C (peak temperature). The TGA result (FIG. 4-2) shows nearly no weight loss below 120 °C.

**Table 4. XRPD pattern analysis data for the Crystal Form A of the oxalate salt of the compound of Formula I**

| No. | 2θ±0.2 (°) | Relative intensity (%) | No. | 2θ±0.2 (°) | Relative intensity (%) |
|---|---|---|---|---|---|
| 1 | 10.664 | 1.1 | 24 | 33.078 | 4.5 |
| 2 | 13.162 | 30.9 | 25 | 33.972 | 7.6 |
| 3 | 15.072 | 54.8 | 26 | 34.410 | 6.3 |
| 4 | 15.802 | 41.3 | 27 | 35.834 | 0.8 |
| 5 | 16.368 | 4.3 | 28 | 36.498 | 1.4 |
| 6 | 17.025 | 5.8 | 29 | 37.253 | 3.7 |
| 7 | 17.833 | 100.0 | 30 | 37.884 | 2.2 |
| 8 | 19.498 | 89.8 | 31 | 39.048 | 6.9 |
| 9 | 20.323 | 14.8 | 32 | 39.819 | 1.9 |
| 10 | 21.088 | 23.8 | 33 | 41.584 | 2.0 |
| 11 | 23.168 | 35.6 | 34 | 42.535 | 2.3 |
| 12 | 23.492 | 5.1 | 35 | 43.926 | 2.3 |
| 13 | 24.677 | 24.0 | 36 | 44.701 | 2.7 |
| 14 | 25.586 | 7.6 | 37 | 45.045 | 2.3 |
| 15 | 26.481 | 20.1 | 38 | 46.978 | 2.1 |
| 16 | 27.082 | 12.2 | 39 | 47.440 | 1.7 |
| 17 | 28.122 | 1.5 | 40 | 50.257 | 2.1 |
| 18 | 28.805 | 13.3 | 41 | 52.146 | 1.5 |
| 19 | 30.276 | 6.3 | 42 | 52.635 | 2.9 |
| 20 | 30.595 | 8.5 | 43 | 54.631 | 0.6 |
| 21 | 31.631 | 4.0 | 44 | 56.595 | 0.7 |
| 22 | 32.002 | 9.4 | 45 | 59.341 | 0.7 |
| 23 | 32.226 | 15.2 | | | |

### Example 6. Crystal Form A of Mucate Salt of Compound of Formula I

100 mg of the compound of Formula I and 1.1 equivalents of mucic acid were weighed out and placed into a 10 mL vial, and a certain amount of isopropyl acetate was added. The mixture was stirred overnight at room temperature. The reaction solution was filtered, and the filter cake was washed and dried to give the Crystal Form A of the mucate salt of the compound of Formula I (143 mg, chemical purity: 99.4%). The XRPD pattern (FIG. 5-1) shows relatively high crystallinity. The characteristic peak positions are shown in Table 5. The DSC result (FIG. 5-2) shows an endothermic signal at 171.5 °C (peak temperature).

**Table 5. XRPD pattern analysis data for the Crystal Form A of the mucate salt of the compound of Formula I**

| No. | 2θ±0.2 (°) | Relative intensity (%) | No. | 2θ±0.2 (°) | Relative intensity (%) |
|---|---|---|---|---|---|
| 1 | 11.489 | 1.6 | 23 | 26.852 | 20.8 |
| 2 | 12.975 | 9.1 | 24 | 28.326 | 3.2 |
| 3 | 13.451 | 9.0 | 25 | 29.702 | 12.7 |
| 4 | 14.739 | 15.5 | 26 | 30.170 | 4.5 |
| 5 | 15.310 | 10.2 | 27 | 30.758 | 47.9 |
| 6 | 16.171 | 38.7 | 28 | 33.087 | 5.2 |
| 7 | 17.231 | 9.9 | 29 | 34.460 | 17.1 |
| 8 | 17.743 | 12.8 | 30 | 34.869 | 10.9 |
| 9 | 18.123 | 15.9 | 31 | 36.684 | 11.7 |
| 10 | 18.470 | 9.6 | 32 | 37.671 | 26.2 |
| 11 | 18.974 | 4.1 | 33 | 39.007 | 2.4 |
| 12 | 19.647 | 100.0 | 34 | 39.782 | 4.3 |
| 13 | 19.845 | 6.8 | 35 | 40.310 | 4.1 |
| 14 | 20.491 | 2.6 | 36 | 40.929 | 7.1 |
| 15 | 21.511 | 23.3 | 37 | 44.052 | 5.7 |
| 16 | 22.194 | 3.9 | 38 | 48.770 | 2.0 |
| 17 | 22.794 | 32.1 | 39 | 49.356 | 2.1 |
| 18 | 23.556 | 7.1 | 40 | 51.922 | 1.7 |
| 19 | 23.976 | 2.4 | 41 | 53.392 | 1.7 |
| 20 | 24.551 | 7.2 | 42 | 55.290 | 2.6 |
| 21 | 24.854 | 9.7 | 43 | 56.844 | 1.6 |
| 22 | 26.080 | 7.0 | | | |

### Example 7. Crystal Form A of Hydrobromide Salt of Compound of Formula I

200 mg of the compound of Formula I and 3.3 equivalents of hydrobromic acid were weighed out and placed into a 10 mL vial, and a certain amount of isopropanol was added. The mixture was stirred overnight at room temperature. The reaction solution was filtered, and the filter cake was washed and dried to give the Crystal Form A of the hydrobromide salt of the compound of Formula I (280 mg). The XRPD pattern (FIG. 6-1) shows relatively high crystallinity. The characteristic peak positions are shown in Table 6. The DSC result (FIG. 6-2) shows an endothermic signal at 284.1 °C (peak temperature). The TGA result (FIG. 6-2) shows no weight loss below 120 °C.

**Table 6. XRPD pattern analysis data for the Crystal Form A of the hydrobromide salt of the compound of Formula I**

| No. | 2θ±0.2 (°) | Relative intensity (%) | No. | 2θ±0.2 (°) | Relative intensity (%) |
|---|---|---|---|---|---|
| 1 | 8.551 | 2.8 | 31 | 34.321 | 7.6 |
| 2 | 11.515 | 100.0 | 32 | 34.819 | 6.1 |
| 3 | 14.871 | 19.3 | 33 | 35.487 | 3.5 |
| 4 | 16.026 | 1.0 | 34 | 36.362 | 2.3 |
| 5 | 16.925 | 17.1 | 35 | 37.035 | 2.4 |
| 6 | 17.475 | 59.9 | 36 | 37.474 | 2.3 |
| 7 | 18.385 | 1.0 | 37 | 38.075 | 4.3 |
| 8 | 19.359 | 25.6 | 38 | 39.085 | 9.1 |
| 9 | 20.010 | 2.0 | 39 | 39.444 | 2.6 |
| 10 | 21.082 | 10.8 | 40 | 40.177 | 2.4 |
| 11 | 21.462 | 9.3 | 41 | 40.417 | 2.8 |
| 12 | 21.974 | 13.3 | 42 | 42.023 | 9.9 |
| 13 | 23.011 | 4.1 | 43 | 42.897 | 6.3 |
| 14 | 23.566 | 23.2 | 44 | 43.082 | 4.3 |
| 15 | 24.012 | 21.6 | 45 | 43.998 | 1.0 |
| 16 | 24.881 | 59.3 | 46 | 44.549 | 3.1 |
| 17 | 25.246 | 5.1 | 47 | 44.812 | 5.5 |
| 18 | 25.592 | 2.5 | 48 | 45.346 | 1.7 |
| 19 | 26.205 | 65.0 | 49 | 46.554 | 3.0 |
| 20 | 27.458 | 13.9 | 50 | 46.896 | 3.8 |
| 21 | 27.977 | 14.9 | 51 | 47.666 | 1.3 |
| 22 | 28.452 | 22.4 | 52 | 48.308 | 4.2 |
| 23 | 29.060 | 1.4 | 53 | 48.693 | 6.8 |
| 24 | 29.718 | 28.4 | 54 | 49.596 | 1.7 |
| 25 | 30.282 | 4.5 | 55 | 50.705 | 1.7 |
| 26 | 30.526 | 5.9 | 56 | 51.662 | 1.8 |
| 27 | 31.123 | 17.3 | 57 | 52.392 | 5.0 |
| 28 | 32.375 | 13.6 | 58 | 53.894 | 5.5 |
| 29 | 33.365 | 6.3 | 59 | 56.611 | 1.4 |
| 30 | 33.60 | 4.6 | 60 | 58.624 | 1.3 |

### Example 8. Crystal Form A of Fumarate Salt of Compound of Formula I

100 mg of the compound of Formula I and 1.1 equivalents of fumaric acid were weighed out and placed into a 10 mL vial, and a certain amount of isopropanol was added. The mixture was stirred overnight at room temperature. The reaction solution was filtered, and the filter cake was washed and dried to give the Crystal Form A of the fumarate salt of the compound of Formula I (150 mg, chemical purity: 100%). The XRPD pattern (FIG. 7-1) shows relatively high crystallinity. The characteristic peak positions are shown in Table 7. The DSC result (FIG. 7-2) shows an endothermic signal at 191.9 °C (peak temperature). The TGA result (FIG. 7-2) shows nearly no weight loss below 120 °C.

**Table 7. XRPD pattern analysis data for the Crystal Form A of the fumarate salt of the compound of Formula I**

| No. | 2θ±0.2 (°) | Relative intensity (%) | No. | 2θ±0.2 (°) | Relative intensity (%) |
|---|---|---|---|---|---|
| 1 | 5.201 | 0.6 | 13 | 21.435 | 100.0 |
| 2 | 10.588 | 14.3 | 14 | 22.550 | 8.0 |
| 3 | 12.491 | 2.8 | 15 | 23.386 | 19.0 |
| 4 | 13.403 | 10.7 | 16 | 23.565 | 15.9 |
| 5 | 14.031 | 38.1 | 17 | 25.598 | 2.7 |
| 6 | 15.720 | 24.2 | 18 | 26.064 | 1.6 |
| 7 | 16.342 | 27.8 | 19 | 26.664 | 3.5 |
| 8 | 17.135 | 1.9 | 20 | 28.712 | 3.5 |
| 9 | 19.802 | 3.7 | 21 | 29.566 | 1.9 |
| 10 | 20.213 | 14.9 | 22 | 36.881 | 2.3 |
| 11 | 20.889 | 50.3 | 23 | 40.309 | 3.3 |
| 12 | 20.927 | 53.6 | 24 | 40.339 | 3.0 |

### Example 9. Crystal Form B of Fumarate Salt of Compound of Formula I

100 mg of the compound of Formula I and 1.1 equivalents of fumaric acid were weighed out and placed into a 10 mL vial, and a certain amount of isopropyl acetate was added. The mixture was stirred overnight at room temperature. The reaction solution was filtered, and the filter cake was washed and dried to give the Crystal Form B of the fumarate salt of the compound of Formula I (127 mg, chemical purity: 99.5%). The XRPD pattern (FIG. 8-1) shows relatively high crystallinity. The characteristic peak positions are shown in Table 8. The DSC result (FIG. 8-2) shows endothermic signals at 172.1 °C and 189.8 °C (peak temperatures). The TGA result (FIG. 8-2) shows nearly no weight loss below about 120 °C.

**Table 8. XRPD pattern analysis data for the Crystal Form B of the fumarate salt of the compound of Formula I**

| No. | 20+0.2 (°) | Relative intensity (%) | No. | 2θ±0.2 (°) | Relative intensity (%) |
|---|---|---|---|---|---|
| 1 | 7.698 | 2.4 | 28 | 25.453 | 84.0 |
| 2 | 10.026 | 2.4 | 29 | 26.055 | 12.1 |
| 3 | 10.723 | 3.9 | 30 | 26.588 | 35.4 |
| 4 | 12.504 | 52.5 | 31 | 27.589 | 23.9 |
| 5 | 13.233 | 13.4 | 32 | 28.295 | 6.7 |
| 6 | 14.084 | 13.1 | 33 | 28.771 | 16.3 |
| 7 | 14.689 | 53.6 | 34 | 29.536 | 17.2 |
| 8 | 15.041 | 33.4 | 35 | 30.329 | 8.2 |
| 9 | 15.688 | 31.3 | 36 | 31.073 | 8.2 |
| 10 | 16.052 | 60.8 | 37 | 31.988 | 15.0 |
| 11 | 16.612 | 45.1 | 38 | 32.629 | 3.1 |
| 12 | 17.462 | 76.1 | 39 | 33.348 | 6.0 |
| 13 | 17.792 | 6.5 | 40 | 34.008 | 4.8 |
| 14 | 18.204 | 4.4 | 41 | 34.676 | 6.7 |
| 15 | 19.106 | 14.7 | 42 | 35.235 | 3.3 |
| 16 | 19.747 | 93.7 | 43 | 36.678 | 5.0 |
| 17 | 20.109 | 32.4 | 44 | 37.956 | 16.7 |
| 18 | 20.911 | 82.4 | 45 | 39.371 | 7.9 |
| 19 | 21.720 | 17.6 | 46 | 39.519 | 8.7 |
| 20 | 22.029 | 28.8 | 47 | 40.747 | 8.8 |
| 21 | 22.430 | 100.0 | 48 | 40.993 | 9.7 |
| 22 | 22.824 | 11.1 | 49 | 42.068 | 2.4 |
| 23 | 23.454 | 17.8 | 50 | 42.643 | 5.8 |
| 24 | 23.800 | 23.4 | 51 | 43.852 | 6.6 |
| 25 | 24.011 | 31.2 | 52 | 44.534 | 2.6 |
| 26 | 24.632 | 46.9 | 53 | 45.006 | 2.7 |
| 27 | 25.046 | 38.9 | 54 | 49.652 | 2.6 |

### Example 10. Crystal Form A of L-Tartrate Salt of Compound of Formula I

100 mg of the compound of Formula I and 1.1 equivalents of L-tartaric acid were weighed out and placed into a 10 mL vial, and a certain amount of isopropyl acetate was added. The mixture was stirred overnight at room temperature. The reaction solution was filtered, and the filter cake was washed and dried to give the Crystal Form A of the L-tartrate salt of the compound of Formula I (127 mg, chemical purity: 100%). The XRPD pattern (FIG. 9-1) shows relatively high crystallinity. The characteristic peak positions are shown in Table 9. The DSC result (FIG. 9-2) shows endothermic signals at 113.6 °C, 136.8 °C, and 202.8 °C (peak temperatures). The TGA result (FIG. 9-2) shows a weight loss of 1.1% at about 70 °C.

**Table 9. XRPD pattern analysis data for the Crystal Form A of the L-tartrate salt of the compound of Formula I**

| No. | 2θ±0.2 (°) | Relative intensity (%) | No. | 2θ±0.2 (°) | Relative intensity (%) |
|---|---|---|---|---|---|
| 1 | 6.148 | 1.4 | 23 | 27.650 | 13.7 |
| 2 | 12.107 | 73.5 | 24 | 28.888 | 37.5 |
| 3 | 12.962 | 41.7 | 25 | 29.718 | 8.8 |
| 4 | 13.686 | 14.4 | 26 | 31.290 | 7.7 |
| 5 | 14.614 | 8.5 | 27 | 35.012 | 19.4 |
| 6 | 16.817 | 100.0 | 28 | 35.925 | 19.8 |
| 7 | 17.082 | 60.0 | 29 | 36.767 | 2.4 |
| 8 | 17.924 | 25.9 | 30 | 37.559 | 21.7 |
| 9 | 18.137 | 33.2 | 31 | 38.931 | 10.3 |
| 10 | 19.137 | 23.9 | 32 | 39.157 | 9.9 |
| 11 | 19.494 | 75.2 | 33 | 39.567 | 4.0 |
| 12 | 20.731 | 22.4 | 34 | 40.308 | 2.0 |
| 13 | 21.967 | 33.0 | 35 | 42.113 | 7.9 |
| 14 | 22.675 | 35.2 | 36 | 42.984 | 3.8 |
| 15 | 23.076 | 18.1 | 37 | 43.087 | 4.7 |
| 16 | 23.839 | 48.5 | 38 | 43.616 | 7.4 |
| 17 | 24.135 | 16.6 | 39 | 44.241 | 3.6 |
| 18 | 24.767 | 35.4 | 40 | 44.988 | 3.1 |
| 19 | 25.346 | 7.3 | 41 | 45.981 | 2.4 |
| 20 | 26.220 | 16.0 | 42 | 49.560 | 2.3 |
| 21 | 26.901 | 55.9 | 43 | 50.804 | 3.8 |
| 22 | 27.240 | 20.9 | 44 | 53.774 | 2.0 |

### Example 11. Crystal Form A of Gallate Salt of Compound of Formula I

100 mg of the compound of Formula I and 1.1 equivalents of gallic acid were weighed out and placed into a 10 mL vial, and a certain amount of methyl tert-butyl ether was added. The mixture was stirred overnight at room temperature. The reaction solution was filtered, and the filter cake was washed and dried to give the Crystal Form A of the gallate salt of the compound of Formula I (137 mg, chemical purity: 98.8%). The XRPD pattern (FIG. 10-1) shows relatively high crystallinity. The characteristic peak positions are shown in Table 10. The DSC result (FIG. 10-2) shows endothermic signals at 119.3 °C, 183.9 °C, and 233.4 °C (peak temperatures). The TGA result (FIG. 10-2) shows a weight loss of 3.7% at about 140 °C.

**Table 10. XRPD pattern analysis data for the Crystal Form A of the gallate salt of the compound of Formula I**

| No. | 2θ±0.2 (°) | Relative intensity (%) | No. | 2θ±0.2 (°) | Relative intensity (%) |
|---|---|---|---|---|---|
| 1 | 6.992 | 1.7 | 20 | 23.480 | 93.2 |
| 2 | 6.964 | 1.4 | 21 | 24.012 | 23.8 |
| 3 | 10.508 | 3.2 | 22 | 24.414 | 38.4 |
| 4 | 10.879 | 15.4 | 23 | 24.978 | 12.3 |
| 5 | 11.529 | 11.4 | 24 | 25.609 | 14.4 |
| 6 | 12.126 | 7.4 | 25 | 26.181 | 43.8 |
| 7 | 12.966 | 8.6 | 26 | 27.498 | 36.1 |
| 8 | 13.689 | 13.9 | 27 | 27.850 | 18.9 |
| 9 | 14.571 | 80.3 | 28 | 30.833 | 3.9 |
| 10 | 15.170 | 71.0 | 29 | 31.485 | 9.8 |
| 11 | 15.469 | 35.9 | 30 | 32.745 | 11.9 |
| 12 | 16.567 | 58.7 | 31 | 34.343 | 10.8 |
| 13 | 17.325 | 48.7 | 32 | 36.981 | 4.7 |
| 14 | 18.178 | 10.1 | 33 | 37.634 | 5.2 |
| 15 | 18.933 | 14.0 | 34 | 38.501 | 4.5 |
| 16 | 20.016 | 44.8 | 35 | 39.582 | 8.0 |
| 17 | 20.569 | 100.0 | 36 | 41.383 | 4.3 |
| 18 | 21.770 | 22.8 | 37 | 42.560 | 4.7 |
| 19 | 22.297 | 10.8 | | | |

### Example 12. Crystal Form A of p-Toluenesulfonate Salt of Compound of Formula I

500 mg of the compound of Formula I and 1.1 equivalents of p-toluenesulfonic acid were weighed out and placed into a 10 mL vial, and a certain amount of isopropanol was added. The mixture was heated to 60 °C (bath temperature) and stirred for 1 h. The reaction solution was cooled to room temperature and filtered, and the filter cake was washed and dried to give the Crystal Form A of the p-toluenesulfonate salt of the compound of Formula I (620 mg, chemical purity: 99.9%). The XRPD pattern (FIG. 11-1) shows relatively high crystallinity. The characteristic peak positions are shown in Table 11. The DSC result (FIG. 11-2) shows an endothermic signal at 189.2 °C (peak temperature). The TGA result (FIG. 11-2) shows nearly no weight loss below about 120 °C.

**Table 11. XRPD pattern analysis data for the Crystal Form A of the p-toluenesulfonate salt of the compound of Formula I**

| No. | 20±0.2 (°) | Relative intensity (%) | No. | 2θ±0.2 (°) | Relative intensity (%) |
|---|---|---|---|---|---|
| 1 | 7.845 | 26.8 | 12 | 21.798 | 4.2 |
| 2 | 12.391 | 5.0 | 13 | 22.473 | 9.0 |
| 3 | 13.526 | 24.2 | 14 | 23.534 | 23.5 |
| 4 | 14.080 | 20.9 | 15 | 24.627 | 12.9 |
| 5 | 15.692 | 12.0 | 16 | 25.368 | 19.6 |
| 6 | 16.451 | 11.2 | 17 | 27.328 | 18.6 |
| 7 | 17.124 | 100.0 | 18 | 28.484 | 3.9 |
| 8 | 17.794 | 11.2 | 19 | 30.907 | 9.3 |
| 9 | 19.848 | 34.4 | 20 | 33.173 | 4.1 |
| 10 | 20.209 | 40.0 | 21 | 37.646 | 4.2 |
| 11 | 20.780 | 71.9 | | | |

### Example 13. Crystal Form A of Hydrochloride Salt of Compound of Formula I

500 mg of the compound of Formula I was weighed out and dissolved in 4 mL of methanol, and 5 equivalents of a solution of hydrochloric acid in dioxane (4 mol/L) was then added dropwise. After the addition, the mixture was stirred at room temperature for 1 h, then heated to 50 °C (bath temperature) and stirred for 1 h, and then cooled to room temperature. The mixture was filtered, and the filter cake was washed and dried to give the hydrochloride salt of the compound of Formula I (150 mg, chemical purity: 99.8%). Then, the product was characterized. The XRPD pattern (FIG. 12-1) shows relatively high crystallinity. The characteristic peak positions are shown in Table 12. The DSC result (FIG. 12-2) shows no endothermic peak below 250 °C and an exothermic signal at 278.4 °C (peak temperature). The TGA result (FIG. 12-2) shows nearly no weight loss below about 120 °C.

**Table 12. XRPD pattern analysis data for the Crystal Form A of the hydrochloride salt of the compound of Formula I**

| No. | 2θ±0.2 (°) | Relative intensity (%) | No. | 2θ±0.2 (°) | Relative intensity (%) |
|---|---|---|---|---|---|
| 1 | 11.893 | 100.0 | 12 | 25.609 | 72.3 |
| 2 | 15.339 | 19.2 | 13 | 26.987 | 23.4 |
| 3 | 16.680 | 26.1 | 14 | 28.243 | 25.4 |
| 4 | 17.444 | 20.9 | 15 | 28.931 | 11.0 |
| 5 | 17.676 | 14.4 | 16 | 31.150 | 14.0 |
| 6 | 19.959 | 6.9 | 17 | 33.375 | 9.9 |
| 7 | 21.356 | 13.8 | 18 | 34.384 | 8.4 |
| 8 | 21.821 | 10.5 | 19 | 37.873 | 5.3 |
| 9 | 22.389 | 20.4 | 20 | 38.934 | 3.1 |
| 10 | 24.529 | 6.4 | 21 | 40.651 | 6.3 |
| 11 | 25.024 | 19.3 | | | |

### Test Example 1. In Vitro Evaluation of Intrasynaptic and Extrasynaptic Functional Activities in Cells

1. Test materials: cells: the cAMP Hunter^{™} CHO-K1 TAAR1 Gs cell line
Assay reagent: HitHunter cAMP Assay Detection Kit
Experimental instrument: PerkinElmer Envision^{™} chemiluminescence signal detector
2. Test method:
   Cell treatment: The cAMP Hunter cell strain was cultured at 37 °C with 5% CO₂ to 70-80% confluence. The cells were harvested, and the cell density was adjusted to 1.5 × 10⁴ cells/mL. The cells were seeded in a white-wall 384-well microplate at a volume of 20 µL, and the plate was incubated overnight at 37 °C with 5% CO₂. The cell culture medium was discarded, and 15 µL of 2:1 HBSS/10 mM Hepes:cAMP XS + Ab reagent was added to each well. The mother solution of the sample was serially diluted with an assay buffer to obtain a 4× sample solution, and 5 µL of the 4× sample solution was added to each well. The plate was incubated at 37 °C or room temperature for 30 or 60 min. 20 µL of cAMP XS + ED/CL mixed lysis buffer was added to each well, and the plate was incubated for 1 h. 20 µL of cAMP XS + EA reagent was added to each well, and the plate was incubated at room temperature for 3 h. The chemiluminescence signals were read using a PerkinElmer Envision^{™} detector.
3. Data analysis:
   EC₅₀ was calculated using CBIS data analysis software (ChemInnovation, CA). The percent activity was calculated using the following formula: % activity = 100% × (test sample average RLU - solvent control average RLU)/(maximum control average RLU - solvent control average RLU). The EC₅₀ of the compound of Formula I was 0.062 µM, indicating good TAAR1 agonistic activity.

### Test Example 2. Pharmacokinetic Study in Rats

Rats: SD rats weighing 200-250 g
Reagents: acetonitrile, MERCK; methanol, MERCK; formic acid, Sigma; tolbutamide, Sigma.
Instruments: water purification equipment, Millipore; electronic balance, METTLER TOLEDO; high-speed benchtop centrifuge, Thermo; water-bath thermostatic shaker, Shanghai Yiheng Technical Co., Ltd.; vortex shaker, Thermo; liquid chromatography-mass spectrometry, AB SCIEX.
Method: SD rats were dosed intravenously (IV) and intragastrically (IG), with 3 SD rats in each group. The compound of Formula I was administered, with DMSO and Tween 80 as vehicles. Administration doses: 2 mg/kg for intravenous administration and 5 mg/kg for intragastric administration. Whole blood samples were collected at 0, 5 min (intravenous), 15 min, 30 min, 1 h, 2 h, 4 h, 6 h, 8 h, 12 h, and 24 h, respectively, placed into heparinized EP tubes, and centrifuged at 13,500 rpm for 10 min to separate plasma. After pretreatment, the plasma samples were analyzed by LC-MS/MS to determine the concentration of the test compound in the plasma samples. The results are shown in Table 13:

**Table 13. Pharmacokinetic parameters of the compound of Formula I after intravenous and intragastric administration to SD rats**

| Subject | IV 2mg/kg | IG 5mg/kg |
|---|---|---|
| Tₘₐₓ(h) | / | 0.417±0.144 |
| Cₘₐₓ or C₀ (nM) | 7541±2278 | 3730±846 |
| AUCₗₐₛₗ(h* nM) | 5971±642 | 9945±1213 |
| T_{1/2} (h) | 1.76±0.344 | 1.85±0.217 |
| Vss_obs(L/kg) | 2.91±0.68 | / |
| Cl_obs(L/h/kg) | 1.73±0.195 | / |
| MRTₗₐₛₜ (h) | 1.60±0.203 | 2.33±0.465 |
| F(%) | / | 66.6±8.12 |

The results show that the compound of Formula I exhibited relatively high exposure and bioavailability after intravenous and intragastric administration to rats.

### Test Example 3. Pharmacodynamic Study in Mice

Mice: C57 mice weighing 20-22 g.
Reagent: normal saline.
Instruments: electronic balance, METTLER TOLEDO; TopScan monitoring system.

3.1. Model preparation: MK-801 can induce overactivity in mice and is commonly used for preclinical evaluations of antipsychotics. The modeling method in this test was as follows: Each mouse was given a single injection of MK-801 intraperitoneally (dose: 0.8 mg/kg).

3.2. Experimental groups: The mice were randomly divided into a model group and a "compound of Formula I" group, with 10 animals in each group. See Table 14 for details.

**Table 14. Animal grouping and dosing**

| Group | Drug type | Administration dose (mg/kg) | Drug concentration (mg/mL) | Route of administration | Administration volume (mL/kg) | Animal Number |
|---|---|---|---|---|---|---|
| Model group | -- | -- | -- | -- | -- | 10 |
| "Compound of Formula I" group | Compound of Formula I | 10 | 1 | Intragastric | 10 | 10 |

3.3. Test method: The mice were randomly divided into the groups. At the start of the test, a single dose of the test sample was administered intragastrically according to Table 14, and the mice were then placed in an activity chamber. After 30 min, each mouse was injected intraperitoneally with MK-801 (0.8 mg/kg) for modeling and returned to the activity chamber. The movement distance (mm) of each mouse after modeling (i.e., within 30-60 min of administering the drug) was recorded and analyzed using a TopScan monitoring system.

3.4. Observation of indexes:
The movement distance (mm) of each mouse after modeling (within 30-60 min of administering the drug) was recorded and analyzed using a TopScan monitoring system. The total movement distance of each mouse after modeling (within 30-60 min of administering the drug) was recorded.

3.5. Statistical analysis:
Data were analyzed and processed using Graphpad 5.0 software. An ANOVA test was used to compare the total movement distance within 30-60 min of each treatment group and with that of the model group. All tests were bilateral tests. p < 0.05 indicates that the difference is statistically significant.

3.6. Test results:
The test results show that the total movement distance of the animals in the "compound of Formula I" group after modeling was 6493.12±6228.87 mm; compared to the total movement distance of the animals in the model group after modeling (63052.14±21618.80 mm), there was a significant reduction (p < 0.05).

3.7. Test conclusion:
Under the experimental conditions, the compound of Formula I (10 mg/kg) exhibited a good inhibitory effect on MK801-induced overactivity.

### Test Example 4. Stability Study

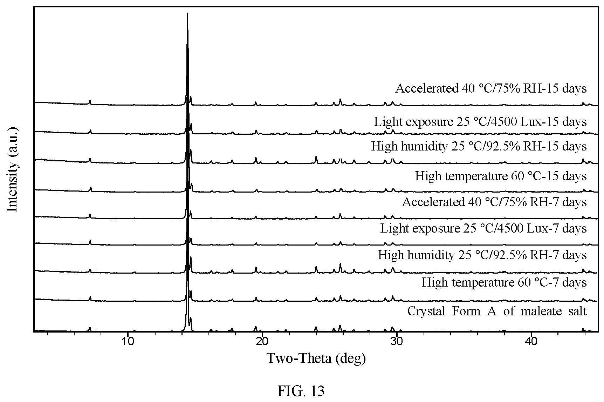

A stability study of the Crystal Form A of the maleate salt of the compound of Formula I was conducted under high temperature (60 °C), high humidity (25 °C/92.5% RH), light exposure (25 °C/4500 Lux), and accelerated (40 °C/75% RH) conditions. Samples were taken for XRPD characterization and HPLC analysis at 7 and 15 days, respectively. The results are shown in Table 15 and FIG. 13. The XRPD results show that the Crystal Form A of the maleate salt remained stable for 15 days under high temperature, high humidity, light exposure, and accelerated conditions, and there was no crystal form transformation. The HPLC results show that the chemical purity of the Crystal Form A of the maleate salt did not change significantly after 15 days of standing under high temperature, high humidity, light exposure, and accelerated conditions; a slight yellowing of the sample was observed after 15 days of standing under light exposure.

**Table 15. The results of a stability study of the Crystal Form A of the maleate salt of the compound of Formula I**

| Crystal form | Conditions | 7 days | | 15 days | |
|---|---|---|---|---|---|
| | | XRPD result | Purity/% | XRPD result | Purity/% |
| Crystal form A of maleate salt of compound of Formula I (Purity: 99.95%) | High temperature 60 °C | Crystal form A | 99.93 | Crystal form A | 99.89 |
| | High humidity 25 °C/92.5% RH | Crystal form A | 99.93 | Crystal form A | 99.90 |
| | Light exposure 25 °C/4500 Lux | Crystal form A, a slight yellowing of the sample | 99.85 | Crystal form A, a slight yellowing of the sample | 99.79 |
| | Accelerated 40 °C/75% RH | Crystal form A | 99.94 | Crystal form A | 99.91 |

## Claims

1. A pharmaceutically acceptable salt of a compound of Formula I, wherein the pharmaceutically acceptable salt is selected from the group consisting of citrate, succinate, maleate, fumarate, L-tartrate, gallate, mucate, phosphate, hydrochloride, oxalate, hydrobromide, sulfate, acetate, DL-lactate, ascorbate, hippurate, p-toluenesulfonate, and diphosphate, preferably maleate, mucate, phosphate, and oxalate:

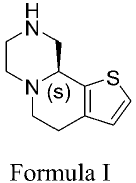

2. A Crystal Form A of a maleate salt of a compound of Formula I, wherein the Crystal Form A of the maleate salt has, in an X-ray powder diffraction pattern using Cu-Kα radiation, characteristic diffraction peaks at the following 20 angles: 14.49±0.2°, 14.72±0.2°, 16.26±0.2°, 17.81±0.2°, 19.58±0.2°, 24.03±0.2°, 25.84±0.2°, and 29.72±0.2°, preferably characteristic diffraction peaks at the following 2θ angles: 7.24±0.2°, 10.56±0.2°, 14.49±0.2°, 14.72±0.2°, 16.26±0.2°, 17.81±0.2°, 19.58±0.2°, 24.03±0.2°, 25.84±0.2°, and 29.72±0.2°; most preferably, the X-ray powder diffraction pattern using Cu-Kα radiation is as shown in FIG. 1-1.

3. A Crystal Form A of a phosphate salt of a compound of Formula I, wherein the Crystal Form A of the phosphate salt has, in an X-ray powder diffraction pattern using Cu-Kα radiation, characteristic diffraction peaks at the following 20 angles: 15.068±0.2°, 15.331±0.2°, 16.011±0.2°, 17.333±0.2°, 17.605±0.2°, 20.119±0.2°, 20.870±0.2°, 23.039±0.2°, 25.159±0.2°, 25.910±0.2°, 26.680±0.2°, and 27.843±0.2°, preferably characteristic diffraction peaks at the following 20 angles: 15.068±0.2°, 15.331±0.2°, 16.011±0.2°, 17.333±0.2°, 17.605±0.2°, 20.119±0.2°, 20.870±0.2°, 21.161±0.2°, 23.039±0.2°, 25.159±0.2°, 25.910±0.2°, 26.680±0.2°, 27.843±0.2°, 28.681±0.2°, 34.958±0.2°, and 35.302±0.2°; preferably, the X-ray powder diffraction pattern using Cu-Kα radiation is as shown in FIG. 2-1.

4. A Crystal Form A of a diphosphate salt of a compound of Formula I, wherein the Crystal Form A of the diphosphate salt has, in an X-ray powder diffraction pattern using Cu-Kα radiation, characteristic diffraction peaks at the following 2θ angles: 11.029±0.2°, 12.607±0.2°, 14.362±0.2°, 14.849±0.2°, 15.635±0.2°, 18.139±0.2°, 19.187±0.2°, 20.687±0.2°, 21.808±0.2°, 22.432±0.2°, 22.715±0.2°, 23.294±0.2°, 23.533±0.2°, 24.283±0.2°, 24.788±0.2°, and 28.295±0.2°, preferably characteristic diffraction peaks at the following 20 angles: 7.839±0.2°, 11.029±0.2°, 12.607±0.2°, 14.362±0.2°, 14.849±0.2°, 15.635±0.2°, 17.454±0.2°, 18.139±0.2°, 19.187±0.2°, 19.773±0.2°, 20.369±0.2°, 20.687±0.2°, 21.049±0.2°, 21.808+0.2°, 22.432±0.2°, 22.715±0.2°, 23.294±0.2°, 23.533±0.2°, 24.283±0.2°, 24.788±0.2°, and 28.295±0.2°; more preferably, the X-ray powder diffraction pattern using Cu-Kα radiation is as shown in FIG. 3-1.

5. A Crystal Form A of an oxalate salt of a compound of Formula I, wherein the Crystal Form A of the oxalate salt has, in an X-ray powder diffraction pattern using Cu-Kα radiation, characteristic diffraction peaks at the following 2θ angles: 13.162±0.2°, 15.072±0.2°, 15.802±0.2°, 17.833±0.2°, 19.498±0.2°, 21.088±0.2°, 23.168±0.2°, 24.677±0.2°, 26.481±0.2°, 28.805±0.2°, and 32.226±0.2°, preferably characteristic diffraction peaks at the following 2θ angles: 13.162±0.2°, 15.072±0.2°, 15.802±0.2°, 17.833±0.2°, 19.498±0.2°, 20.323±0.2°, 21.088±0.2°, 23.168±0.2°, 24.677±0.2°, 26.481±0.2°, 27.082±0.2°, 28.805±0.2°, 32.002±0.2°, and 32.226±0.2°; more preferably, the X-ray powder diffraction pattern using Cu-Kα radiation is as shown in FIG. 4-1.

6. A Crystal Form A of a mucate salt of a compound of Formula I, wherein the Crystal Form A of the mucate salt has, in an X-ray powder diffraction pattern using Cu-Kα radiation, characteristic diffraction peaks at the following 20 angles: 12.975±0.2°, 13.451±0.2°, 14.739±0.2°, 15.310±0.2°, 16.171±0.2°, 17.231±0.2°, 17.743±0.2°, 18.123±0.2°, 18.470±0.2°, 19.647±0.2°, 21.511±0.2°, 22.794±0.2°, 24.854±0.2°, 26.852±0.2°, 29.702±0.2°, 30.758±0.2°, 34.460±0.2°, 34.869±0.2°, 36.684±0.2°, and 37.671±0.2°; more preferably, the X-ray powder diffraction pattern using Cu-Kα radiation is as shown in FIG. 5-1.

7. A Crystal Form A of a hydrobromide salt of a compound of Formula I, wherein the Crystal Form A of the hydrobromide salt has, in an X-ray powder diffraction pattern using Cu-Kα radiation, characteristic diffraction peaks at the following 2θ angles: 11.515±0.2°, 14.871±0.2°, 16.925±0.2°, 17.475±0.2°, 19.359±0.2°, 21.082±0.2°, 21.462±0.2°, 21.974±0.2°, 23.566±0.2°, 24.012±0.2°, 24.881±0.2°, 26.205±0.2°, 27.458±0.2°, 27.977±0.2°, 28.452±0.2°, 29.718±0.2°, 31.123±0.2°, 32.375±0.2°, 39.085±0.2°, and 42.023±0.2°; preferably, the X-ray powder diffraction pattern using Cu-Kα radiation is as shown in FIG. 6-1.

8. A Crystal Form A of a fumarate salt of a compound of Formula I, wherein the Crystal Form A of the fumarate salt has, in an X-ray powder diffraction pattern using Cu-Kα radiation, characteristic diffraction peaks at the following 20 angles: 10.588±0.2°, 13.403±0.2°, 14.031±0.2°, 15.720±0.2°, 16.342±0.2°, 20.889±0.2°, 20.927±0.2°, 21.435±0.2°, and 23.386±0.2°, preferably characteristic diffraction peaks at the following 2θ angles: 10.588±0.2°, 13.403±0.2°, 14.031±0.2°, 15.720±0.2°, 16.342±0.2°, 20.213±0.2°, 20.889±0.2°, 20.927±0.2°, 21.435±0.2°, 23.386±0.2°, and 23.565±0.2°; more preferably, the X-ray powder diffraction pattern using Cu-Kα radiation is as shown in FIG. 7-1.

9. A Crystal Form B of a fumarate salt of a compound of Formula I, wherein the Crystal Form B of the fumarate salt has, in an X-ray powder diffraction pattern using Cu-Kα radiation, characteristic diffraction peaks at the following 20 angles: 12.504±0.2°, 14.689±0.2°, 15.041±0.2°, 15.688±0.2°, 16.052±0.2°, 16.612±0.2°, 17.462±0.2°, 19.747±0.2°, 20.109±0.2°, 20.911±0.2°, 22.029±0.2°, 22.430±0.2°, 23.454±0.2°, 23.800±0.2°, 24.011±0.2°, 24.632±0.2°, 25.046±0.2°, 25.453±0.2°, 26.588±0.2°, and 27.589±0.2°; preferably, the X-ray powder diffraction pattern using Cu-Kα radiation is as shown in FIG. 8-1.

10. A Crystal Form A of an L-tartrate salt of a compound of Formula I, wherein the Crystal Form A of the L-tartrate salt has, in an X-ray powder diffraction pattern using Cu-Kα radiation, characteristic diffraction peaks at the following 2θ angles: 12.107±0.2°, 12.962±0.2°, 16.817±0.2°, 17.082±0.2°, 17.924±0.2°, 18.137±0.2°, 19.137±0.2°, 19.494±0.2°, 20.731±0.2°, 21.967±0.2°, 22.675±0.2°, 23.839±0.2°, 24.767±0.2°, 26.220±0.2°, 26.901±0.2°, 27.240±0.2°, 28.888±0.2°, and 37.559±0.2°; preferably, the X-ray powder diffraction pattern using Cu-Kα radiation is as shown in FIG. 9-1.

11. A Crystal Form A of a gallate salt of a compound of Formula I, wherein the Crystal Form A of the gallate salt has, in an X-ray powder diffraction pattern using Cu-Kα radiation, characteristic diffraction peaks at the following 2θ angles: 10.879±0.2°, 14.571±0.2°, 15.170±0.2°, 15.469±0.2°, 16.567±0.2°, 17.325±0.2°, 20.016±0.2°, 20.569±0.2°, 21.770±0.2°, 23.480±0.2°, 24.012±0.2°, 24.414±0.2°, 26.181±0.2°, 27.498±0.2°, and 27.850±0.2°; preferably, the X-ray powder diffraction pattern using Cu-Kα radiation is as shown in FIG. 10-1.

12. A Crystal Form A of a p-toluenesulfonate salt of a compound of Formula I, wherein the Crystal Form A of the p-toluenesulfonate salt has, in an X-ray powder diffraction pattern using Cu-Kα radiation, characteristic diffraction peaks at the following 20 angles: 7.845±0.2°, 13.526±0.2°, 14.080±0.2°, 17.124±0.2°, 19.848±0.2°, 20.209±0.2°, 20.780±0.2°, 23.534±0.2°, 25.368±0.2°, and 27.328±0.2°; preferably, the X-ray powder diffraction pattern using Cu-Kα radiation is as shown in FIG. 11-1.

13. A Crystal Form A of a hydrochloride salt of a compound of Formula I, wherein the Crystal Form A of the hydrochloride salt has, in an X-ray powder diffraction pattern using Cu-Kα radiation, characteristic diffraction peaks at the following 2θ angles: 11.893±0.2°, 15.339±0.2°, 16.680±0.2°, 17.444±0.2°, 22.389±0.2°, 25.024±0.2°, 25.609±0.2°, 26.987±0.2°, and 28.243±0.2°, preferably characteristic diffraction peaks at the following 2θ angles: 11.893±0.2°, 15.339±0.2°, 16.680±0.2°, 17.444±0.2°, 17.676±0.2°, 21.356±0.2°, 21.821±0.2°, 22.389±0.2°, 25.024±0.2°, 25.609±0.2°, 26.987±0.2°, 28.243±0.2°, 28.931±0.2°, and 31.150±0.2°; more preferably, the X-ray powder diffraction pattern using Cu-Kα radiation is as shown in FIG. 12-1.

14. A pharmaceutical composition, comprising the pharmaceutically acceptable salt according to claim 1 or the crystal form according to any one of claims 2-13, and a pharmaceutically acceptable carrier.

15. Use of the pharmaceutically acceptable salt according to claim 1, the crystal form according to any one of claims 2-13, or the pharmaceutical composition according to claim 14 in the preparation of a TAAR1 agonist medicament.

16. Use of the pharmaceutically acceptable salt according to claim 1, the crystal form according to any one of claims 2-13, or the pharmaceutical composition according to claim 14 in the preparation of a medicament for preventing and/or treating central nervous system-related diseases or disorders and cardiovascular or metabolic diseases.

17. The use according to claim 16, wherein the central nervous system diseases or disorders include schizophrenia, schizophrenia spectrum disorder, acute schizophrenia, chronic schizophrenia, NOS schizophrenia, psychotic disorder, schizoid personality disorder, schizotypal personality disorder, delusional disorder, psychosis, mental disorder, brief psychotic disorder, shared psychotic disorder, psychotic disorder due to a physical disease, drug-induced psychosis, psychoaffective disorder, aggressive delirium, Parkinson's psychosis, excitative psychosis, Tourette syndrome, organic or NOS psychosis, epilepsy, epileptic seizures, agitation, post-traumatic stress disorder, behavioral disorder, neurodegenerative disease, Alzheimer's disease, Parkinson's disease, dyskinesias, Huntington's disease, dementia, affective disorder, anxiety, affective psychosis, obsessive compulsive disorder, vertigo, pain, fibromyalgia, migraine, cognitive impairment, movement disorder, restless leg syndrome, multiple sclerosis, psychoactive substance abuse, and stress-related disorders.

18. The use according to claim 17, wherein the affective psychosis includes depression, major depressive disorder and dysthymia, bipolar disorder, bipolar depression, manic disorder, seasonal affective psychosis, attention deficit disorder (ADD), and attention deficit hyperactivity disorder (ADHD); the pain includes neuropathic pain, neuropathic pain susceptibility state, and inflammatory pain; the stress-related disorders include: acute stress disorder, post-traumatic stress disorder, and adjustment disorder.

19. The use according to claim 16, wherein the cardiovascular or metabolic diseases include diabetes, diabetic complications, obesity, dyslipidemia, and hypertension.
